# EUROPEAN PATENT SPECIFICATION

(11) **EP 4 233 689 B1**
(45) Date of publication and mention of the grant of the patent: **06.05.2026**
(21) Application number: 21882681.6
(22) Date of filing: 13.10.2021
(51) Int. Cl.: A61B 3/10, A61B 3/00, A61B 3/14, A61B 3/13

(54) **OPHTHALMOLOGICAL OBSERVATION DEVICE, OPHTHALMOLOGICAL IMAGE PROCESSING METHOD, AND RECORDING MEDIUM**
OPHTHALMOLOGISCHE BEOBACHTUNGSVORRICHTUNG, OPHTHALMOLOGISCHES BILDVERARBEITUNGSVERFAHREN UND AUFZEICHNUNGSMEDIUM
DISPOSITIF D'OBSERVATION OPHTALMOLOGIQUE, MÉTHODE DE TRAITEMENT D'IMAGE OPHTALMOLOGIQUE, ET SUPPORT D'ENREGISTREMENT

(30) Priority: 23.10.2020 JP 2020177703
(43) Date of publication of application: 30.08.2023
(73) Proprietor: TOPCON CORPORATION, Tokyo 174-8580 (JP)
(72) Inventor: YAMADA, Kazuhiro, Tokyo 174-8580 (JP)
(74) Representative: Gagel, Roland
(86) International application number: PCT/JP2021/037930
(87) International publication number: WO 2022/085540

(56) References cited:
- WO-A1-2019/240257
- JP-A- 2005 103 030
- JP-A- 2013 169 309
- JP-A- 2013 198 719
- JP-A- 2020 000 678
- US-A1- 2004 189 937
- US-A1- 2012 076 471
- US-A1- 2019 099 226

## Description

### [TECHNICAL FIELD]

The present disclosure relates generally to an ophthalmic observation apparatus, an ophthalmic image processing method, and a recording medium.

### [BACKGROUND OF THE INVENTION]

An ophthalmic observation apparatus is an apparatus for observing an eye of a patient (which will be referred to as a subject's eye hereinafter). Ophthalmic observation is conducted to grasp the condition of the subject's eye in various situations such as examination, surgery, and treatment.

Conventional ophthalmic observation apparatuses are configured to provide a user with a magnified image formed by an objective lens, a variable magnification optical system, etc. via an eyepiece. In recent years, some ophthalmic observation apparatuses are configured to photograph a magnified image formed by an objective lens, a variable magnification optical system, etc. with an image sensor, and display the photographed image obtained (such an ophthalmic observation apparatus will be referred to as an ophthalmic observation apparatus of the first aspect). Examples of such ophthalmic observation apparatuses include slit lamp microscopes, surgical microscopes, and fundus cameras (retinal cameras). In addition, various kinds of ophthalmic examination apparatuses such as refractometers, keratometers, tonometers, specular microscopes, wavefront analyzers, and microperimeters are also provided with the function of the ophthalmic observation apparatus of the first aspect.

Furthermore, some ophthalmic observation apparatuses of recent years use optical scanning (such an ophthalmic observation apparatus will be referred to as an ophthalmic observation apparatus of the second aspect). Examples of such ophthalmic observation apparatuses include scanning laser ophthalmoscopes (SLOs), and optical coherence tomography (OCT) apparatuses.

Generally, an ophthalmic observation apparatus is configured to provide a moving image of a subject's eye to a user (e.g., a health professional (health care practitioner) such as a doctor). A typical ophthalmic observation apparatus of the first aspect is configured to perform photographing of a moving image using infrared light and/or visible light as illumination light, and real-time display of the moving image obtained by the moving image photography. On the other hand, a typical ophthalmic observation apparatus of the second aspect is configured to perform data collection (data acquisition) by repetitive optical scanning, real-time image reconstruction based on datasets sequentially collected, and real-time moving image display of images sequentially reconstructed. The real-time moving image provided in these ways is called an observation image.

Image quality adjustment is required for providing a good observation image. However, desired image quality differs from user to user, and also differs depending on the types and phases of examination or surgery. For example, some doctors may prefer an image with a reddish tint, while others may prefer an image with a greenish tint. In addition, cataract surgery, which is one of the most common type of ophthalmic surgery, involves the phases (processes, steps) of alignment, incision creation, ocular viscoelastic agent injection, continuous curvilinear capsulorhexis (CCC), phacoemulsification aspiration, lens cortex aspiration, intraocular lens (IOL) insertion, IOL centering, ocular viscoelastic agent removal, and incision closure. However, image quality desired by doctors may vary depending on the phases of cataract surgery. Further, there are cases in which a doctor wants to selectively enhance the quality of the image of a site of interest. In addition, desired image quality may differ depending on the conditions or states of the subject's eye.

While desired quality of observation images varies as described above, image quality adjustment of typical conventional ophthalmic observation apparatuses has been manually conducted each time needed. Such manual adjustment is very complicated and time-consuming, and has been one of the factors that have caused the lengthening of the time required for examination and/or surgery. On the other hand, it may be conceivable to automatically perform image quality adjustment. However, considering the large variety of desired image quality, the adjustment has to be done manually after all.

[PATENT DOCUMENT 1] Japanese Unexamined Patent Application Publication No. 2003-310556
[PATENT DOCUMENT 2] Japanese Unexamined Patent Application Publication No. 2009-118955

WO 2019/240257 A1 discloses a medical image processing device which is configured to perform processing of an input image, e.g. of a subject's eye, using several image quality enhancement engines created by machine learning with different learning data to generate a plurality of high quality images, and to display the high quality images.

US 2019/099226 A1 discloses an ophthalmic system comprising a surgical suite which processes and displays a variety of distinct surgical views, e.g. a view that focuses on a macula, a view that focuses on a retinal tear, etc.

### [BRIEF SUMMARY OF THE INVENTION]

An object of the present disclosure is to provide a new technique for facilitating ophthalmic observation.

The invention is set out in the appended set of claims.

### [BRIEF DESCRIPTION OF THE SEVERAL VIEWS OF THE DRAWING]

FIG. 1 is a schematic diagram illustrating an example of the configuration of the ophthalmic observation apparatus (ophthalmic surgical microscope) according to the embodiment example.
FIG. 2 is a schematic diagram illustrating an example of the configuration of the ophthalmic observation apparatus according to the embodiment example.
FIG. 3 is a schematic diagram illustrating an example of the configuration of the ophthalmic observation apparatus according to the embodiment example.
FIG. 4 is a schematic diagram illustrating an example of the configuration of the ophthalmic observation apparatus according to the embodiment example.
FIG. 5 is a schematic diagram illustrating an example of the configuration of the ophthalmic observation apparatus according to the embodiment example.
FIG. 6 is a schematic diagram illustrating an example of the configuration of the ophthalmic observation apparatus according to the embodiment example.
FIG. 7 is a schematic diagram illustrating an example of the configuration of the ophthalmic observation apparatus according to the embodiment example.
FIG. 8 is a schematic diagram illustrating an example of the configuration of the ophthalmic observation apparatus according to the embodiment example.
FIG. 9 is a schematic diagram illustrating an example of the configuration of the ophthalmic observation apparatus according to the embodiment example.
FIG. 10 is a schematic diagram illustrating an example of the configuration of the ophthalmic observation apparatus according to the embodiment example.
FIG. 11 is a flowchart illustrating an example of processing performed by the ophthalmic observation apparatus according to the embodiment example.
FIG. 12A is a schematic diagram for describing an example of processing performed by the ophthalmic observation apparatus according to the embodiment example.
FIG. 12B is a schematic diagram for describing an example of processing performed by the ophthalmic observation apparatus according to the embodiment example.
FIG. 12C is a schematic diagram for describing an example of processing performed by the ophthalmic observation apparatus according to the embodiment example.
FIG. 12D is a schematic diagram for describing an example of processing performed by the ophthalmic observation apparatus according to the embodiment example.
FIG. 12E is a schematic diagram for describing an example of processing performed by the ophthalmic observation apparatus according to the embodiment example.
FIG. 13 is a flowchart illustrating an example of processing performed by the ophthalmic observation apparatus according to the embodiment example.
FIG. 14A is a schematic diagram for describing an example of processing performed by the ophthalmic observation apparatus according to the embodiment example.
FIG. 14B is a schematic diagram for describing an example of processing performed by the ophthalmic observation apparatus according to the embodiment example.
FIG. 14C is a schematic diagram for describing an example of processing performed by the ophthalmic observation apparatus according to the embodiment example.
FIG. 14D is a schematic diagram for describing an example of processing performed by the ophthalmic observation apparatus according to the embodiment example.
FIG. 14E is a schematic diagram for describing an example of processing performed by the ophthalmic observation apparatus according to the embodiment example.
FIG. 14F is a schematic diagram for describing an example of processing performed by the ophthalmic observation apparatus according to the embodiment example.
FIG. 14G is a schematic diagram for describing an example of processing performed by the ophthalmic observation apparatus according to the embodiment example.
FIG. 15 is a schematic diagram illustrating an example of the configuration of the ophthalmic observation apparatus according to the embodiment example.
FIG. 16 is a schematic diagram illustrating an example of the configuration of the ophthalmic observation apparatus according to the embodiment example.
FIG. 17 is a schematic diagram illustrating an example of the configuration of an ophthalmic image processing apparatus not claimed in this patent application.

### [DETAILED DESCRIPTION OF THE INVENTION]

Some aspect examples of an ophthalmic observation apparatus, an ophthalmic image processing method, and a recording medium according to some embodiments will be described in detail with reference to the drawings. It should be noted that any of the matters and items described in the documents cited in the present disclosure and any known techniques and technologies may be combined with any of the aspect examples.

The ophthalmic observation apparatus according to some aspect examples is used in medical practice (healthcare practice) such as examination, surgery, and treatment of the subject's eye, in order to grasp (understand, recognize, find) the state of the subject's eye. The ophthalmic observation apparatus of the aspect examples described herein is mainly a surgical microscope system. However, ophthalmic observation apparatuses of embodiments are not limited to surgical microscope systems. For example, the ophthalmic observation apparatus of some aspect examples may be any of a slit lamp microscope, a fundus camera, a refractometer, a keratometer, a tonometer, a specular microscope, a wavefront analyzer, a microperimeter, an SLO, and an OCT apparatus. Also, the ophthalmic observation apparatus of some aspect examples may be a system that includes any one or more of these apparatus examples. More generally, the ophthalmic observation apparatus of some aspect examples may be any type of ophthalmic apparatus having an observation function.

A target ocular site for observation (ocular site to be observed, ocular site subject to observation) by using the ophthalmic observation apparatus may be any site of the subject's eye, and may be any site of the anterior segment and/or any site of the posterior segment. Examples of the observation target sites of the anterior segment include cornea, iris, anterior chamber, corner angle, crystalline lens, ciliary body, and zonule of Zinn. Examples of the observation target sites of the posterior segment include retina, choroid, sclera, and vitreous body. The observation target site is not limited to tissues of an eye ball, and may be any site subject to be observed in ophthalmic medical practice (and/or medical practice in other medical fields) such as eyelid, meibomian gland, and orbit (eye socket, eye pit).

At least one or more of the functions of the elements described in the present disclosure are implemented by using a circuit configuration (or circuitry) or a processing circuit configuration (or processing circuitry). The circuitry or the processing circuitry includes any of the followings, all of which are configured and/or programmed to execute at least one or more functions disclosed herein: a general purpose processor, a dedicated processor, an integrated circuit, a central processing unit (CPU), a graphics processing unit (GPU), an application specific integrated circuit (ASIC), a programmable logic device (e.g., a simple programmable logic device (SPLD), a complex programmable logic device (CPLD), or a field programmable gate array (FPGA)), a conventional circuit configuration or circuitry, and any combination of these. A processor is considered to be processing circuitry or circuitry that includes a transistor and/or another circuitry. In the present disclosure, circuitry, a unit, a means, or a term similar to these is hardware that executes at least one or more functions disclosed herein, or hardware that is programmed to execute at least one or more functions disclosed herein. Hardware may be the hardware disclosed herein, or alternatively, known hardware that is programmed and/or configured to execute at least one or more functions described herein. In the case where the hardware is a processor, which may be considered as a certain type of circuitry, then circuitry, a unit, a means, or a term similar to these is a combination of hardware and software. In this case, the software is used to configure the hardware and/or the processor.

### < Ophthalmic observation apparatus >

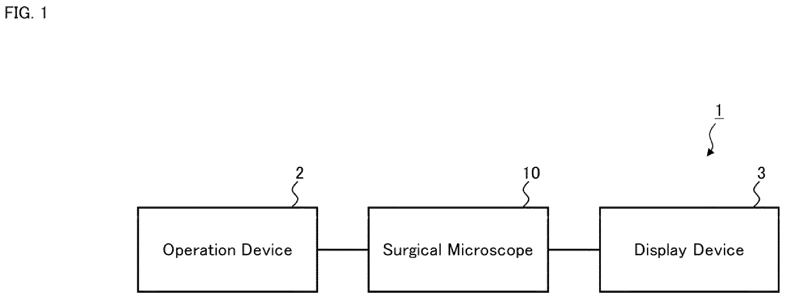

FIG. 1 shows the configuration of the ophthalmic observation apparatus of some aspect examples.

The ophthalmic observation apparatus 1 (surgical microscope system, operation microscope system) according to the present embodiment includes the operation device 2 and the display device 3. According to the invention as defined in claim 1, it includes the surgical microscope (operation microscope) 10. In some aspects, the surgical microscope 10 may include at least one of the operation device 2 and the display device 3. In some aspects, the display device 3 may not be included in the ophthalmic observation apparatus 1. In other words, the display device 3 may be a peripheral device of the ophthalmic observation apparatus 1.

### < Operation device 2 >

The operation device 2 includes an operation device and/or an input device. For example, the operation device 2 may include any of a button, a switch, a mouse, a keyboard, a trackball, an operation panel, a dial, and the like. Typically, the operation device 2 includes a foot switch, like standard (general, normal, usual) ophthalmic surgical microscopes.

### < Display device 3 >

The display device 3 displays an image of the subject's eye acquired by the surgical microscope 10. The display device 3 includes a display device such as a flat panel display. The display device 3 may include any of various kinds of display devices such as a touch panel. The display device 3 of some typical aspects includes a display device with a large screen. The display device 3 includes one or more display devices. In the case where the display device 3 includes two or more display devices, for example, one may be a display device with a relatively large screen and one of the other(s) may be a display device with a relatively small screen.

The operation device 2 and the display device 3 do not have to be separate devices. For example, a device having both the operation function and the display function, such as a touch panel, may be used as the display device 3. In such a case, the operation device 2 may include a computer program in addition to the touch panel. A content of an operation made by the operation device 2 is sent to a processor (not shown in the drawings) as an electric signal. Further, a graphical user interface (GUI) displayed on the display device 3 and the operation device 2 may be used to conduct operations (instructions) and input information. In some aspects, the functions of the operation device 2 and the display device 3 may be implemented with a touch screen.

### < Surgical microscope 10 >

The surgical microscope 10 is used for observation of the eye of a patient (subject's eye) in the supine position. The surgical microscope 10 performs photographing of the subject's eye to generate digital image data. In particular, the surgical microscope 10 generates a moving image of the subject's eye. The moving image (video, movie) generated by the surgical microscope 10 is transmitted to the display device 3 through a wired and/or wireless signal path and displayed on the display device 3. The user (e.g., surgeon) can carry out surgery while observing the subject's eye through the displayed image. In addition to such observation through the displayed image, the surgical microscope 10 of some aspects may be capable of providing observation through an eyepiece as in the past.

In some aspects, the surgical microscope 10 includes a communication device for transmitting and receiving electrical signals to and from the operation device 2. The operation device 2 receives an operation (instruction) performed by the user and generates an electric signal (operation signal) corresponding to the operation. The operation signal is transmitted to the surgical microscope 10 through a wired and/or wireless signal path. The surgical microscope 10 executes processing corresponding to the operation signal received.

### < Optical system of surgical microscope 10 >

An example of the configuration of the optical system of the surgical microscope 10 will be described. Below, directions are defined as follows, for convenience of description: the z direction is defined to be the optical axis direction (direction along the optical axis) of the objective lens (the z direction is, for example, the vertical direction, the up and down direction during surgery); the x direction is defined to be a predetermined direction perpendicular to the z direction (the x direction is, for example, the horizontal direction during surgery, and the left and right direction for the surgeon and the patient during surgery); and the y direction is defined to be the direction perpendicular to both the z and x directions (the y direction is, for example, the horizontal direction during surgery, the front and back direction for the surgeon during surgery, and the body axis direction (direction along the body axis) for the patient during surgery).

In addition, the case where the observation optical system includes a pair of left and right optical systems (optical systems capable of binocular observation) will be mainly described below. However, an observation optical system of some other aspects may have an optical system for monocular observation, and it will be understood by those skilled in the art that the configuration described below may be incorporated into the aspects for monocular observation.

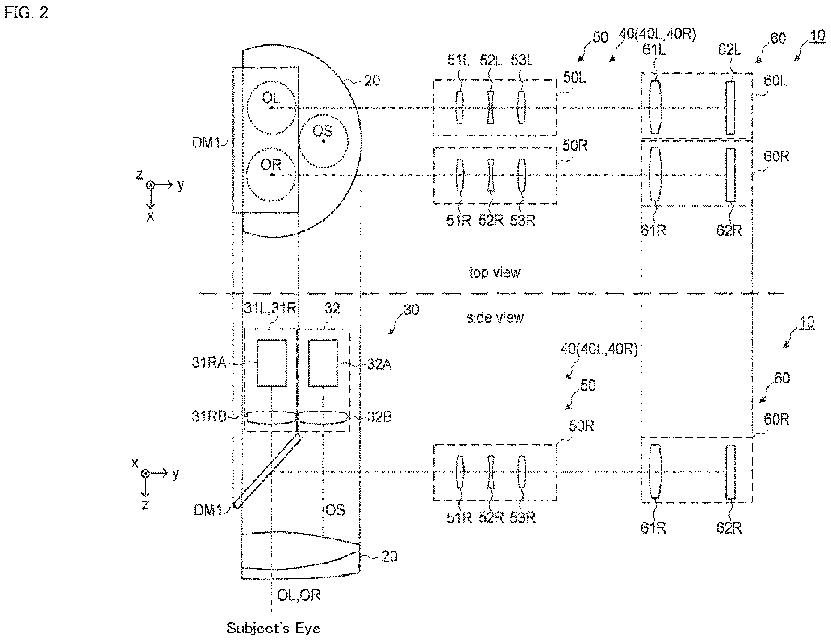

FIG. 2 shows an example of the configuration of the optical system of the surgical microscope 10. FIG. 2 illustrates a schematic top view of the optical system viewed from above and a schematic side view of the optical system viewed from the side in association with each other. In order to simplify the illustration, the illumination optical system 30 arranged above the objective lens 20 is omitted in the top view.

The surgical microscope 10 includes the objective lens 20, the dichroic mirror DM1, the illumination optical system 30, and the observation optical system 40. The observation optical system 40 includes the zoom expander 50 and the imaging camera 60. In some aspects, the illumination optical system 30 or the observation optical system 40 includes the dichroic mirror DM1.

The objective lens 20 is arranged to face the subject's eye. The objective lens 20 is arranged such that its optical axis is oriented along the z direction. The objective lens 20 may include two or more lenses.

The dichroic mirror DM1 couples the optical path of the illumination optical system 30 and the optical path of the observation optical system 40 with each other. The dichroic mirror DM1 is arranged between the illumination optical system 30 and the objective lens 20. The dichroic mirror DM1 transmits illumination light from the illumination optical system 30 and directs the illumination light to the subject's eye through the objective lens 20. Also, the dichroic mirror DM1 reflects return light from the subject's eye incident through the objective lens 20 and directs the return light to the imaging camera 60 of the observation optical system 40.

The dichroic mirror DM1 coaxially couples the optical path of the illumination optical system 30 and the optical path of the observation optical system 40 with each other. In other words, the optical axis of the illumination optical system 30 and the optical axis of the observation optical system 40 intersect at the dichroic mirror DM1. In the case where the illumination optical system 30 includes an illumination optical system for left eye (31L) and an illumination optical system for right eye (31R) and where the observation optical system 40 includes an observation optical system for left eye (40L) and an observation optical system for right eye (40R), the dichroic mirror DM1 coaxially couples the optical path of the illumination optical system for left eye (the first illumination optical system 31L) and the optical path of the observation optical system for left eye (40L) with each other, and coaxially couples the optical path of the illumination optical system for right eye (the first illumination optical system 31R) and the optical path of the observation optical system for right eye (40R) with each other.

The illumination optical system 30 is an optical system for illuminating the subject's eye through the objective lens 20. The illumination optical system 30 may be configured to selectively illuminate the subject's eye with two or more pieces of illumination light having different color temperatures. The illumination optical system 30 projects illumination light having a designated color temperature onto the subject's eye under the control of a controller (the controller 200 described later).

The illumination optical system 30 includes the first illumination optical systems 31L and 31R and the second illumination optical system 32.

Each of the optical axis OL of the first illumination optical system 31L and the optical axis OR of the first illumination optical system 31R is arranged with the optical axis of the objective lens 20 in a substantially coaxial manner. Such arrangements enable an illumination mode referred to as "0-degree illumination" and therefore make it possible to obtain a red reflex image (transillumination image) formed by utilizing diffuse reflection from eye fundus. The present aspect allows the red reflex image of the subject's eye to be observed with both eyes.

The second illumination optical system 32 is arranged in such a manner that its optical axis OS is eccentric (deviated, shifted) from the optical axis of the objective lens 20. The first illumination optical systems 31L and 31R and the second illumination optical system 32 are arranged such that the deviation of the optical axis OS with respect to the optical axis of the objective lens 20 is larger than the deviations of the optical axes OL and OR with respect to the optical axis of the objective lens 20. Such arrangements enable an illumination mode referred to as "angled illumination (oblique illumination)" and therefore enables binocular observation of the subject's eye while preventing ghosting caused by corneal reflection or the like. In addition, the arrangements enable detailed observation of unevenness and irregularities of sites and tissues of the subject's eye.

The first illumination optical system 31L includes the light source 31LA and the condenser lens 31LB. The light source 31LA outputs illumination light having a wavelength in the visible range (visible region) corresponding to color temperature of 3000 K (kelvins), for example. The illumination light emitted from the light source 31LA passes through the condenser lens 31LB, passes through the dichroic mirror DM1, passes through the objective lens 20, and then is incident on the subject's eye.

The first illumination optical system 31R includes the light source 31RA and the condenser lens 31RB. The light source 31RA also outputs illumination light having a wavelength in the visible range corresponding to color temperature of 3000 K, for example. The illumination light emitted from the light source 31RA passes through the condenser lens 31RB, passes through the dichroic mirror DM1, passes through the objective lens 20, and then is incident on the subject's eye.

The second illumination optical system 32 includes the light source 32A and the condenser lens 32B. The light source 32A outputs illumination light having a wavelength in the visible range corresponding to a color temperature within the range of 4000 K to 6000 K, for example. The illumination light emitted from the light source 32A passes through the condenser lens 32B, passes through the objective lens 20 without passing through the dichroic mirror DM1, and then is incident on the subject's eye.

In the present aspect example, the color temperature of the illumination light from the first illumination optical systems 31L and 31R is lower than the color temperature of the illumination light from the second illumination optical system 32. Such a configuration makes it possible to observe the subject's eye in warm colors using the first illumination optical systems 31L and 31R, and therefore enables detailed observation of the structure and morphology of the subject's eye.

In some aspects, each of the optical axes OL and OR is movable relative to the optical axis of the objective lens 20. The direction of the relative movement is a direction that intersects the optical axis of the objective lens 20, and the relative movement is represented by a displacement vector in which at least one of the x component and the y component is not zero. In some aspects, the optical axes OL and OR may be mutually independently movable. On the other hand, in some aspects, the optical axes OL and OR may be integrally movable. For example, the surgical microscope 10 includes a movement mechanism (31d) configured to move the first illumination optical systems 31L and 31R mutually independently or integrally, and therefore the movement mechanism moves the first illumination optical systems 31L and 31R mutually independently or integrally in a direction intersecting the optical axis of the objective lens 20. Such a configuration makes it possible to conduct adjustment of the appearance condition (appearance state) of the subject's eye. In some aspects, the movement mechanism operates under the control of a controller (the controller 200 described later).

In some aspects, the optical axis OS is movable relative to the optical axis of the objective lens 20. The direction of the relative movement is a direction that intersects the optical axis of the objective lens 20, and the relative movement is represented by a displacement vector in which at least one of the x component and the y component is not zero. For example, the surgical microscope 10 includes a movement mechanism (32d) configured to move the second illumination optical system 32, and therefore the movement mechanism moves the second illumination optical system 32 in a direction that intersects the optical axis of the objective lens 20. With such a configuration, it becomes possible to conduct adjustment of the appearance condition (appearance state) of unevenness and irregularities of sites and tissues of the subject's eye. In some aspects, the movement mechanism operates under the control of a controller (the controller 200 described later).

As described above, the present aspect is configured such that the illumination optical system 30 is arranged at the position directly above the objective lens 20 (the position in the transmission direction of the dichroic mirror DM1) and the observation optical system 40 is arranged at the position in the reflection direction of the dichroic mirror DM1. For example, the observation optical system 40 may be arranged such that the angle formed by the optical axis of the observation optical system 40 and the plane perpendicular to the optical axis of the objective lens 20 (the xy plane) belongs to the range between - 20 degrees and + 20 degrees.

According to the configuration of the present aspect, the observation optical system 40, which generally has a longer optical path length than the illumination optical system 30, is arranged substantially parallel to the xy plane. Hence, the observation optical system 40 of the present aspect does not interfere with the surgeon's field of view while conventional surgical microscopes, whose observation optical system is oriented along the vertical direction in front of the surgeon's eyes, does. Therefore, the surgeon is capable of easily seeing the screen of the display device 3 arranged in front of the surgeon. In other words, the visibility of displayed information (images and videos of the subject's eye, and other various kinds reference information) during surgery etc. is improved. In addition, since the housing is not placed in front of the surgeon's eyes, it does not give a sense of oppression to the surgeon, thereby reducing the burden on the surgeon.

The observation optical system 40 is an optical system for observation of an image formed based on return light of the illumination light incident from the subject's eye through the objective lens 20. In the present aspect, the observation optical system 40 guides the image to an image sensor of the imaging camera 60.

As described above, the observation optical system 40 includes the observation optical system for left eye 40L and the observation optical system for right eye 40R. The configuration of the observation optical system for left eye 40L and the configuration of the observation optical system for right eye 40R are the same as or similar to one another. In some aspects, the observation optical system for left eye 40L and the observation optical system for right eye 40R may be configured such that their optical arrangements can be changed independently of each other.

The zoom expander 50 is also referred to as a beam expander, a variable beam expander, or the like. The zoom expander 50 includes the zoom expander for left eye 50L and the zoom expander for right eye 50R. The configuration of the zoom expander for left eye 50L and the configuration of the zoom expander for right eye 50R are the same as or similar to each other. In some aspects, the zoom expander for left eye 50L and the zoom expander for right eye 50R may be configured such that their optical arrangements can be changed independently of each other.

The zoom expander for left eye 50L includes the plurality of zoom lenses 51L, 52L, and 53L. At least one of the zoom lenses 51L, 52L, and 53L is movable in the direction along the optical axis with a variable magnification mechanism (not shown in the drawings).

Similarly, the zoom expander for right eye 50R includes the plurality of zoom lenses 51R, 52R, and 53R, and at least one of the zoom lenses 51R, 52R, and 53R is movable in the direction along the optical axis with a variable magnification mechanism (not shown in the drawings).

The variable magnification mechanism(s) may be configured to move a zoom lens of the zoom expander for left eye 50L and a zoom lens of the zoom expander for right eye 50R mutually independently or integrally in the directions along the optical axes. As a result of this, the magnification ratio for photographing the subject's eye is changed. In some aspects, the variable magnification mechanism(s) operates under the control of a controller (the controller 200 described later).

The imaging camera 60 is a device that photographs an image formed by the observation optical system 40 and generates digital image data. The imaging camera 60 is typically a digital camera (digital video camera). The imaging camera 60 includes the imaging camera for left eye 60L and the imaging camera for right eye 60R. The configuration of the imaging camera for left eye 60L and the configuration of the imaging camera for right eye 60R are the same as or similar to one another. In some aspects, the imaging camera for left eye 60L and the imaging camera for right eye 60R may be configured such that their optical arrangements can be changed independently of each other.

The imaging camera for left eye 60L includes the imaging lens 61L and the image sensor 62L. The imaging lens 61L forms an image based on the return light that has passed through the zoom expander for left eye 50L, on the imaging surface (light receiving surface) of the image sensor 62L. The image sensor 62L is an area sensor, and may typically be a charge-coupled device (CCD) image sensor or a complementary metal oxide semiconductor (CMOS) image sensor. The image sensor 62L operates under the control of a controller (the controller 200 described later).

The imaging camera for right eye 60R includes the imaging lens 61R and the image sensor 62R. The imaging lens 61R forms an image based on the return light that has passed through the zoom expander for right eye 50R, on the imaging surface (light receiving surface) of the image sensor 62R. The image sensor 62R is an area sensor, and may typically be a CCD image sensor or a CMOS image sensor. The image sensor 62R operates under the control of a controller (the controller 200 described later).

### < Processing system >

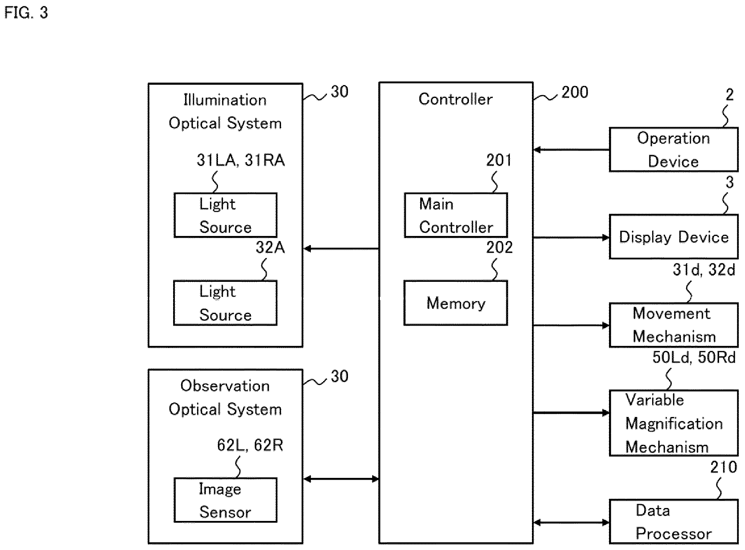

The processing system of the ophthalmic observation apparatus 1 will be described. Some configuration examples of the processing system are shown in FIG. 3 to FIG. 10. Any two or more of the plurality of configuration examples described below may be combined at least in part. Note that the configuration of the processing system is not limited to the examples described below.

The controller 200 executes control of each part of the ophthalmic observation apparatus 1. The controller 200 includes the main controller 201 and the memory 202. The main controller 201 includes a processor and executes control of each part of the ophthalmic observation apparatus 1. For example, the processor may load and run a program stored in the memory 202 or another storage device, thereby implementing a function according to the present aspect. In addition, the processor may use (e.g., referring, processing, calculating, etc.) data and/or information stored in the memory 202 or another storage device in order to implement a function according to the present aspect.

The main controller 201 may control the light sources 31LA, 31RA, and 32A of the illumination optical system 30, the image sensors 62L and 62R of the observation optical system 40, the movement mechanisms 31d and 32d, the variable magnification mechanisms 50Ld and 50Rd, the operation device 2, the display device 3, and other component parts.

Controls of the light source 31LA include turning on and off the light source, adjusting the light amount, adjusting the diaphragm (aperture), and the like. Controls of the light source 31RA include turning on and off the light source, adjusting the light amount, adjusting the diaphragm (aperture), and the like. The main controller 201 may perform mutually exclusive control of the light sources 31LA and 31RA. Controls of the light source 32A include turning on and off the light source, adjusting the light amount, adjusting the diaphragm (aperture), and the like.

In the case where the illumination optical system 30 includes a light source whose color temperature can be varied, the main controller 201 may change the color temperature of emitted illumination light by controlling such a light source.

Controls of the image sensor 62L include exposure adjustment, gain adjustment, photographing rate adjustment, and the like. Controls of the image sensor 62R include exposure adjustment, gain adjustment, photographing rate adjustment, and the like. Further, the main controller 201 may control the image sensors 62L and 62R such that the photographing timings of the image sensors 62L and 62R match each other or such that the difference between the photographing timings of the image sensors 62L and 62R lies within a predetermined time. In addition, the main controller 201 may perform control of loading digital data obtained by the image sensors 62L and 62R.

The movement mechanism 31d moves the light sources 31LA and 31RA mutually independently or integrally in a direction that intersects the optical axis of the objective lens 20. By controlling the movement mechanism 31d, the main controller 201 moves the optical axes OL and OR mutually independently or integrally with respect to the optical axis of the objective lens 20.

The movement mechanism 32d moves the light source 32A independently or integrally in a direction that intersects the optical axis of the objective lens 20. By controlling the movement mechanism 32d, the main controller 201 moves the optical axis OS with respect to the optical axis of the objective lens 20.

In some aspects, the main controller 201 may be configured to control the movement mechanisms 31d and 32d in an interlocking manner.

The variable magnification mechanism 50Ld moves at least one of the plurality of zoom lenses 51L to 53L of the zoom expander for left eye 50L in the optical axis direction (direction along the optical axis). The main controller 201 changes the magnification ratio of the observation optical system for left eye 40L by controlling the variable magnification mechanism 50Ld.

Similarly, the variable magnification mechanism 50Rd moves at least one of the plurality of zoom lenses 51R to 53R of the zoom expander for right eye 50R in the optical axis direction (direction along the optical axis). The main controller 201 changes the magnification ratio of the observation optical system for right eye 40R by controlling the variable magnification mechanism 50Rd.

Controls for the operation device 2 include operation permission control, operation prohibition control, operation signal transmission control and/or operation signal reception control from the operation device 2, and other controls. The main controller 201 receives an operation signal generated by the operation device 2 and executes control corresponding to the operation signal received.

Controls for the display device 3 include information display control and other controls. As a display controller, the main controller 201 displays an image based on digital image data generated by the image sensors 62L and 62R on the display device 3. Typically, the main controller 201 may display a moving image (video, movie) based on digital image data (video signal) generated by the image sensors 62L and 62R on the display device 3. Further, the main controller 201 may display a still image (frame) included in the moving image on the display device 3. In addition, the main controller 201 may display an image (a moving image, a still image, etc.) obtained by processing the digital image data generated by the image sensors 62L and 62R on the display device 3. Furthermore, the main controller 201 may display, on the display device 3, any information generated by the ophthalmic observation apparatus 1, any information acquired from the outside by the ophthalmic observation apparatus 1, and other types of information.

Further, as a display controller, the main controller 201 may create an image for left eye from the digital image data generated by the image sensor 62L and create an image for right eye from the digital image data generated by the image sensor 62R, and then display the created image for left eye and the created image for right eye on the display device 3 in such a manner as to enable stereoscopic vision. For example, the main controller 201 may create a pair of left and right parallax images from the image for left eye and the image for right eye, and display the pair of parallax images on the display device 3. With this, the user (e.g., surgeon) can recognize the pair of parallax images as a stereoscopic image by using a known stereoscopic method or technique. The stereoscopic method applicable to the present aspect may be freely selected, and for example, may be any of the following methods: a stereoscopic method for naked eyes; a stereoscopic method using an auxiliary device (polarized glasses, etc.); a stereoscopic method by applying image processing (image synthesis, image composition, rendering, etc.) to an image for left eye and an image for right eye; a stereoscopic method by displaying a pair of parallax images simultaneously; a stereoscopic method by alternately displaying a pair of parallax images; and a stereoscopic method of a combination of two or more of the above methods.

The data processor 210 executes various kinds of data processes. Some examples of processing that may be executed by the data processor 210 will be described below.

### < Detailed examples of processing system >

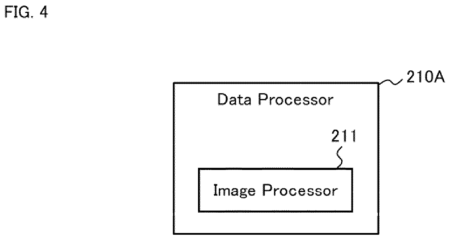

Some examples of processing that may be executed by the data processor 210 will be described together with related elements. FIG. 4 to FIG. 10 show configuration examples of the data processor 210 (and related elements). Any two or more of the configuration examples shown in FIG. 4 to FIG. 10 may be combined at least in part. The data processor 210 (each element thereof) includes a processor that operates on the basis of predetermined software (program), and is implemented by the cooperation of hardware and software.

The data processor 210A shown in FIG. 4 is an example of the data processor 210 of FIG. 3. The data processor 210A of the present example includes the image processor 211. The image processor 211 applies image processing (first image processing) with each of a plurality of different values of a predetermined image parameter, to a still image included in a moving image of the subject's eye generated by the surgical microscope 10 (first moving image, video, movie). With this, a plurality of processed images is created from the still image. The processed image creating process performed by the image processor 211 may be performed in parallel with (concurrently with) the moving image photographing of the subject's eye performed by the surgical microscope 10.

The type of the image parameter used in the first image processing may be freely selected. For example, the image parameter may include any one or more of the types of the parameters exemplified as follows: a color tone parameter (a parameter for color tone conversion); a brightness parameter (a parameter for brightness conversion); a contrast parameter (a parameter for contrast conversion); a gain parameter (a parameter for changing the gain); a gamma parameter (a parameter for gamma correction (correction of response characteristics of image gradation)); a color temperature parameter (a parameter for color temperature conversion); a white balance parameter (a parameter for white balance conversion); an RGB balance parameter (a parameter for balance conversion between R value, G value, and B value); a gray balance parameter (a parameter for gray balance conversion); an edge enhancement parameter (a parameter for edge enhancement); a shadow enhancement parameter (a parameter for shadow enhancement); a sharpening parameter (a parameter for sharpening); and a high dynamic range parameter (a parameter for HDR synthesis). The types of image parameters applicable to the present example are not limited to the types listed above, and may more generally be any parameters that can be used for changing the appearance of a displayed image such as a parameter for display control, a parameter for image representation, a parameter for image correction, a parameter for image adjustment, and the like.

In the first image processing, the image processor 211 performs application of each of a plurality of image processing respectively with a plurality of different values of a single image parameter, to a still image included in the first moving image. For example, the image processor 211 may be configured to apply each of N number of image processing (N number of color tone conversions) respectively with N values of the color tone parameter, to a still image (where N is an integer equal to or greater than 2). As a result of this, N pieces of processed images are created which are represented in mutually different color tones. The same applies to the cases where an image parameter other than the color tone parameter is used.

Further, in the first image processing, the image processor 211 may perform application of each of a plurality of image processing respectively with combinations of a plurality of different values of two image parameters, to a still image included in the first moving image. For example, the image processor 211 may be configured to apply each of N×M number of image processing consisting of combinations of N values of the color tone parameter and M values of the brightness parameter (color tone conversions and brightness conversions), to a still image (where N is an integer equal to or greater than 2 and M is an integer equal to or greater than 2). As a result of this, N×M pieces of processed images are created which are represented by mutually different combinations of color tone and brightness. The same applies to the cases where combinations other than the combination of the color tone parameter and the brightness parameter are used.

Further, the number of image parameters to be combined may be any number equal to or more than two. In addition, it is not necessary to use all of the plurality of values prepared for each image parameter. For example, in the case where N pieces of values of the color tone parameter and M pieces of values of the brightness parameter are prepared, combinations of N1 pieces of values of the color tone parameter and M1 pieces of values of the brightness parameter may be considered. Here, N1 is equal to or smaller than N and M1 is equal to or smaller than M, and further, N1 may be smaller than N and/or M1 may be smaller than M.

The plurality of processed images created in this way is displayed on the display device 3 by the main controller 201. Here, the main controller 201 is configured to display the plurality of processed images themselves created by the image processor 211 on the display device 3, or may display thumbnails (reduced images) of the processed images on the display device 3.

The mode of displaying the plurality of processed images may be freely selected. In some aspects, the main controller 201 may display two or more processed images (or thumbnails thereof) of the plurality of processed images side by side. Here, the main controller 201 may perform a process of displaying the first group selected from the plurality of processed images side by side, and a process of switching from the side-by-side display of the first group to the side-by-side display of the second group in response to reception of a predetermined trigger. The trigger for switching the groups displayed side by side is issued manually or automatically.

In some aspects, the main controller 201 may sequentially display two or more processed images (or thumbnails thereof) of the plurality of processed images. Switching of the displayed processed images is performed manually or automatically.

In the case of displaying thumbnails, the main controller 201 (or, the image processor 211 or the data processor 210A) executes a process of creating thumbnails from the processed images created by the first image processing.

The user (e.g., a surgeon, an assistant who has received an instruction from the surgeon, etc.) may select a desired processed image from a plurality of processed images (or thumbnails thereof) displayed on the display device 3.

The number of processed images selected may be freely selected. In the case where one processed image is selected, the selected processed image is subject to the subsequent process. In the case where two or more processed images are selected, one or more or all of the two or more selected processed images are subject to the subsequent process. For example, the image processor 211A (or the data processor 210A) may be configured to select one processed image from the two or more selected processed images on the basis of a predetermined algorithm.

The ophthalmic observation apparatus 1 includes an image receiving unit that receives an instruction from the user for selecting a processed image. The instruction receiving unit may be freely selected. For example, the user may issue an instruction using the operation device 2. In some aspects, the instruction receiving unit may include any of the following options: a voice recognition unit that detects and recognizes an instruction issued by voice; a line-of-sight recognition unit that detects and recognizes an instruction issued by line-of-sight; a brain wave recognition unit that detects and recognizes an instruction issued by a brain wave; a finger pointing recognition unit that detects and recognizes an instruction issued by finger pointing; and a biological signal recognition unit that detects and recognizes an instruction issued by a biological signal.

Upon the user performing selection of a processed image, the ophthalmic observation apparatus 1 executes processing based on the processed image selected. Some examples of this processing will be described below.

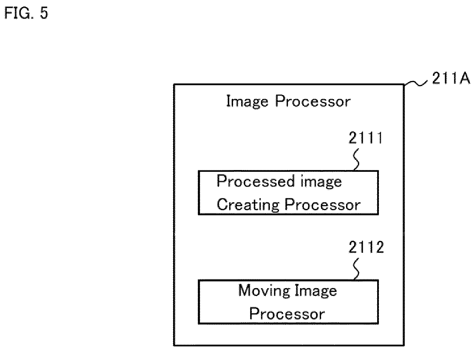

The configuration example shown in FIG. 5 will be described. The image processor 211A shown in FIG. 5 is an example of the image processor 211 shown in FIG. 4. The image processor 211A of the present example includes the processed image creating processor 2111 and the moving image processor 2112.

The processed image creating processor 2111 is configured to execute at least a part of the above processing (at least one of the above processes) described with reference to FIG. 4, for example.

The moving image processor 2112 is configured to execute processing of a moving image based on a processed image selected by the user from the plurality of processed images displayed on the display device 3. For example, the moving image processor 2112 first acquires an image parameter(s) corresponding to the processed image selected by the user. That is, the moving image processor 2112 first acquires the value(s) of the image parameter(s) used in the first image processing for creating the processed image selected by the user. Next, the moving image processor 2112 applies image processing (the second image processing) based on the value(s) of the image parameter(s) to a moving image. The moving image to which the second image processing is applied is at least a moving image generated by the surgical microscope 10 after the selection of the processed image has been made by the user (this moving image is referred to as the second moving image). As a result of this, a moving image can be obtained which has undergone the same image processing as the processed image selected by the user. Note that in the case where one or more still images included in the moving image (first moving image) acquired before the user's selection of the processed image are stored, the same image processing may be applied to at least one of the one or more still images.

The main controller 201 displays the second moving image, to which the second image processing has been applied, on the display device 3. Typically, while performing moving image photography of the subject's eye by the surgical microscope 10, the ophthalmic observation apparatus 1 may perform real-time application of the second image processing to the moving image (the second moving image) acquired by this moving image photography and real-time display of the moving image to which the second image processing has been applied. This allows the user to observe in real time a moving image to which image processing with the same image parameter values as those for the processed image selected by the user has been applied.

As described above, the number of processed images selected by the user may be freely selected. For example, in the case where the number of selected processed images is one, the moving image processor 2112 may apply image processing using the value of the image parameter (one value) corresponding to the selected one processed image to the second moving image as the second image processing.

On the other hand, in the case where the number of selected processed images is two or more, the moving image processor 2112 may select one processed image from the two or more selected processed images and apply image processing, using the value of the image parameter (one value) corresponding to the one processed image selected, to the second moving image as the second image processing. As an alternative example, the moving image processor 2112 may select one value from the two or more values of the image parameters corresponding to the two or more selected processed images, and apply image processing using the selected one value to the second moving image as the second image processing. These selection processes carried out by the moving image processor 2112 are executed on the basis of predetermined algorithms. For example, the moving image processor 2112 may be configured to analyze the two or more processed images selected by the user to calculate a predetermined image quality evaluation value, and select one processed image having the optimum image quality evaluation value. As an alternative example, the moving image processor 2112 may be configured to, in the case where two or more image parameters are used in the first image processing, perform selection of a processed image or selection of an image parameter value(s) with reference to a priority between the mage parameters determined in advance. In this case, for example, the moving image processor 2112 may be configured to select the optimum value (e.g., the highest contrast value) from a plurality of values of an image parameter having the highest priority.

In another aspect where the number of selected processed images is two or more, the moving image processor 2112 may determine one value based on two or more values of image parameters respectively corresponding to the two or more selected processed images, and apply image processing with the determined one value to the second moving image as the second image processing. The arithmetic processing for the determination of one value from the two or more values may be freely selected, and may be statistical calculation, for example. A statistical value calculated by the statistical calculation may be of any kind such as an average value, a median value, a maximum value, a minimum value, or other statistical value. Further, the arithmetic processing to be applied may be determined in advance or may be determined for each processing. As an example of the latter, the moving image processor 2112 may determine the type of the arithmetic processing (statistical calculation) to be applied, based on any one or more of the following exemplary factors: the type of image parameter used in the first image processing; the number of processed images selected by the user; the type of image acquired by the surgical microscope 10; the phase of the medical practice (the phase of surgery in the present example), and other factors.

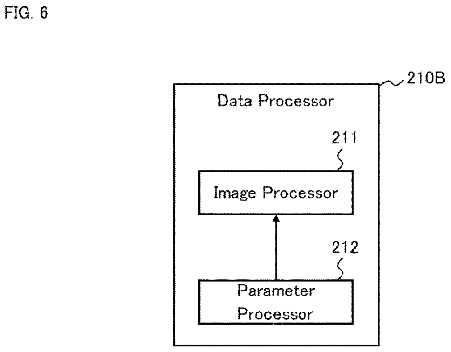

Next, moving onto FIG. 6. The data processor 210B shown in FIG. 6 is an example of the data processor 210 of FIG. 3. The data processor 210B of the present example includes the image processor 211 and the parameter processor 212.

The image processor 211 may be configured to perform the same or similar processing as or to the image processor 211 or the image processor 211A described above.

The parameter processor 212 executes processing related to image parameters. The image processor 211 may perform processing based on an output from the parameter processor 212. For example, the parameter processor 212 is configured to record the value(s) of the image parameter(s) used in the second image processing by the moving image processor 2112 (recording unit), and the image processor 211 is configured to execute another image processing (e.g., another first image processing, another second image processing) using the value of the image parameter recorded by the parameter processor 212. Note that the parameter processor 212 as the recording unit may be configured to record only the value of the image parameter used in the second image processing, or may be configured to record other values in addition to the value of the image parameter used in the second image processing. As an example of the latter, the parameter processor 212 as the recording unit may record any of the following values: the value of the image parameter corresponding to each processed image selected by the user; one or more values of the image parameter corresponding to one or more of a plurality of processed images selected by the user; and the value of the image parameter manually adjusted.

Some examples of the configuration of the parameter processor 212 and some examples of processing executed using the parameter processor 212 will be described below.

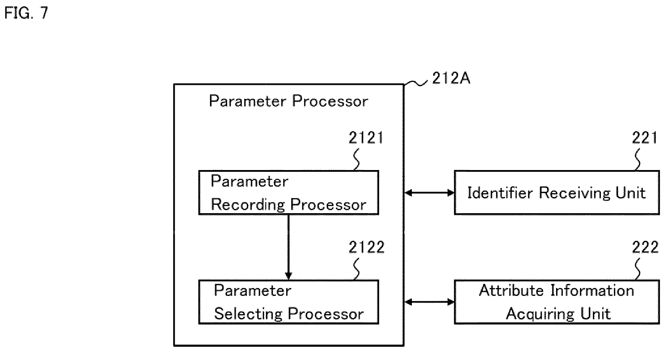

The parameter processor 212A shown in FIG. 7 is an example of the parameter processor 212 of FIG. 6. The parameter processor 212A includes the parameter recording processor 2121 and the parameter selecting processor 2122. In the case where the parameter processor 212A of the present example is employed, the ophthalmic observation apparatus 1 may include the identifier receiving unit 221 and includes the attribute information acquiring unit 222.

The identifier receiving unit 221 receives an identifier of a user (referred to as a user ID). The user is typically a surgeon. The user ID may be represented, for example, by a string of characters or an image (e.g., a barcode, a two dimensional barcode, etc.) assigned to the surgeon (doctor) at a concerned medical institution (medical facility, health facility), the name of the surgeon, and biometric information of the surgeon (e.g., face, fingerprint, palm print, iris pattern, voice, etc.).

The identifier receiving unit 221 may include any device (hardware, software) configured for receiving such user IDs, and may include, for example, the operation device 2, a camera, a barcode scanner, a biometric information scanner, a microphone, a processor, or like devices.

The parameter recording processor 2121 functions as the recording unit described above, and is configured to record a value of an image parameter in association with the user ID received by the identifier receiving unit 221. This makes it possible to identify which user has selected which image parameter value. With this, for example, it becomes possible to execute retrieval of a value of an image parameter using a user ID as a search query.

The attribute information acquiring unit 222 is configured to acquire attribute information indicating an attribute of medical practice for the subject's eye. In other words, the attribute information acquiring unit 222 acquires attribute information indicating various attributes relating to the medical treatment (surgery in the present example) being performed on the subject's eye using the ophthalmic observation apparatus 1.

The attributes of surgery include the types of surgery corresponding to sites subject to surgery, the types of surgery corresponding to diseases, or the like. Examples of the types of surgery corresponding to sites subject to surgery include anterior segment surgery, posterior segment surgery, corneal surgery, corner angle surgery, ciliary body surgery, retinal surgery, vitreous body surgery, and the like. Examples of the type of surgery corresponding to diseases include cataract surgery, glaucoma surgery, corneal transplantation surgery, retinal cell transplantation surgery, retinal detachment surgery, laser photocoagulation, and the like. The same applies to medical practices other than surgery such as examinations, treatments, and screening.

The attributes of medical practices are not limited to the types of medical practices. For example, attribute information may include information indicating a plurality of phases of surgery. For example, the phases of cataract surgery include alignment, incision creation (keratotomy), ocular viscoelastic agent injection, CCC, phacoemulsification aspiration, lens cortex aspiration, IOL insertion, IOL centering, ocular viscoelastic agent removal, and incision closure. Information indicating such a surgical phase may be included in attribute information of some examples. Note that a phase included in attribute information may be determined in advance or may be determined by a surgeon. Further, two or more phases to which the same image parameter value is applied may be grouped together (as a phase group).

Attribute information may be represented, for example, by a string of characters or an image (e.g., a barcode, a two dimensional barcode, etc.) assigned to a medical practice at a concerned medical institution, the name of a medical practice, or the like.

The attribute information acquiring unit 222 may include any device (hardware, software) configured for receiving such attribute information, and may include the operation device 2, a camera, a barcode scanner, a biometric information scanner, a microphone, a processor, or like devices.

In addition, the attribute information acquiring unit 222 may be configured to automatically identify the current phase by analyzing an image (video) generated by the surgical microscope 10, an operation being performed using the operation device 2, an image being displayed on the display device 3, or the like. In the case where the procedure of surgery is determined in advance, the attribute information acquiring unit 222 may be configured to automatically identify the current phase by referring to the stages (processes, steps) having been carried out up to the present stage.

The main controller 201 may be configured to display information indicating the phase automatically identified by the attribute information acquiring unit 222 on the display device 3. Then, the user can judge (check) whether or not the displayed information is correct, and input the result of the judgement using the operation device 2 or other devices. Such a configuration allows the user to check the phase automatically identified by the attribute information acquiring unit 222, thus preventing the wrong phase from being recorded.

The parameter recording processor 2121 functioning as the recording unit records the value of the image parameter in association with the attribute information acquired by the attribute information acquiring unit 222. This makes it possible to identify which image parameter value the user has selected in which medical practice (e.g., in which type or phase of medical practice). For example, it becomes possible to execute searches for image parameter values using the types or phases of surgery as search queries. In addition, the attribute information may include any types or kinds of information such as degrees of disease progression, the dates of performance of medical practices, or skill levels of surgeons.

In the case where the ophthalmic observation apparatus 1 includes both the identifier receiving unit 221 and the attribute information acquiring unit 222, the parameter recording processor 2121 functioning as the recording unit may be configured to record the value of the image parameter in association with both the user ID received by the identifier receiving unit 221 and the attribute information acquired by the attribute information acquiring unit 222. Such a configuration makes it possible to identify which user has selected which image parameter value in which medical practice (e.g., in which type or phase of medical practice). For example, it becomes possible to execute a search for a value of an image parameter using a user ID and/or a type or phase of surgery as a search query.

The parameter selecting processor 2122 is configured to select at least one value from the values of the image parameters recorded in the past by the parameter recording processor 2121. For example, the parameter selecting processor 2122 may be configured to execute selection of a valued of an image parameter by the search query described above.

The image processor 211 (211A) may be configured to apply image processing based on the value of the image parameter selected by the parameter selecting processor 2122 to a moving image generated by the surgical microscope 10 (third moving image). The third moving image may be a moving image of any type, and may be, for example, the second moving image described above, a moving image that has been acquired in another medical practice for the same subject, a moving image that has been acquired in a medical practice for another subject, or the like.

In the case where image parameter value selection is performed using a user ID as a search query, image processing may be applied to the third moving image by reusing a value of an image parameter used in the past by the same user (e.g., the same surgeon). This makes it possible to easily provide the third moving image with the user's preferred display mode (e.g., color, brightness, contrast, etc.).

In the case where image parameter value selection is performed using attribute information of a medical practice as a search query, image processing may be applied to the third moving image by reusing a value of an image parameter used in the past corresponding to the type or phase of a medical practice. This makes it possible to easily provide the third moving image with a display mode (e.g., color, brightness, contrast, etc.) suitable for the type or phase of the medical practice.

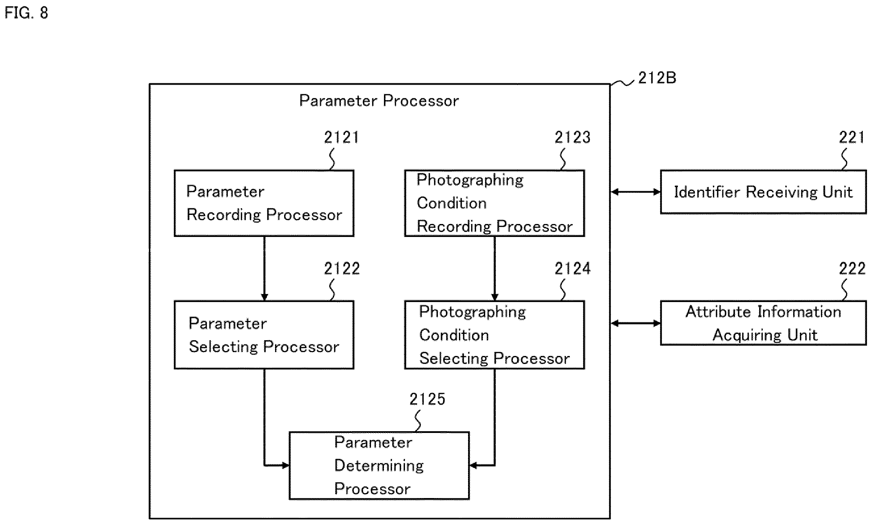

The parameter processor 212B shown in FIG. 8 is an example of the parameter processor 212 of FIG. 6. The parameter processor 212B includes the parameter recording processor 2121 and the parameter selecting processor 2122 as the parameter processor 212A of FIG. 7, and further includes the photographing condition recording processor 2123, the photographing condition selecting processor 2124, and the parameter determining processor 2125. The configuration and the operation of the parameter recording processor 2121 and the configuration and the operation of the parameter selecting processor 2122 may be the same as or similar to those in the case of FIG. 7, and the description thereof will be omitted.

In the case where the parameter processor 212B of the present example is employed, the ophthalmic observation apparatus 1 may include the identifier receiving unit 221 and includes the attribute information acquiring unit 222. The configuration and the operation of the identifier receiving unit 221 and the configuration and the operation of the attribute information acquiring unit 222 may be the same as or similar to those in the case of FIG. 7, and the description thereof will be omitted.

The photographing condition recording processor 2123 is configured to record a photographing condition applied to generation of the second moving image executed by the surgical microscope 10, in association with the value of the image parameter recorded by the parameter recording processor 2121. In the present example, the combination of the parameter recording processor 2121 and the photographing condition recording processor 2123 functions as the recording unit.

The photographing condition may include, for example, an illumination condition, an observation condition, an environmental condition, a subject's eye condition, and the like. The illumination condition includes a condition relating to an element of the illumination optical system 30, and examples of which include a light amount (e.g., output light amounts, output intensities of the light sources 31LA, 31RA, and 32A), an aperture value of a diaphragm, and an illumination mode (e.g., 0 degree illumination, angled illumination). The observation condition includes a condition relating to an element of the observation optical system 40, and examples of which include an aperture value of a diaphragm, a magnification ratio, and control values (e.g., gain) of the image sensors 62L and 62R. the environmental condition includes a condition relating to the environment in which imaging is performed, and examples of which include the brightness of the room in which the surgical microscope 10 is installed. The subject's eye condition includes a condition relating to the subject's eye, and examples of which include the presence or absence of a disease, the type or degree of a disease affected, a pupil diameter, and the degree of opacity of a predetermined site (e.g., cornea, crystalline lens, vitreous body).

Since the present example is configured to record information by both the parameter recording processor 2121 and the photographing condition recording processor 2123, the photographing condition may also be considered in addition to the information considered in the example of FIG. 7.

The photographing condition selecting processor 2124 is configured to select a photographing condition that is associated with the value of the image parameter selected by the parameter selecting processor 2122, from among the photographing conditions recorded in the past by the photographing condition recording processor 2123. As a result of this, in addition to the value of the image parameter selected by the parameter selecting processor 2122, the photographing condition that is associated with the value of the image parameter may also be acquired.

The parameter determining processor 2125 is configured to determine a value of an image parameter based on the value of the image parameter selected by the parameter selecting processor 2122 and the photographing condition selected by the photographing condition selecting processor 2124.

Regarding a controllable condition of the surgical microscope 10 such as the illumination condition and the observation condition, the main controller 201 may control the surgical microscope 10 (e.g., the illumination optical system 30, the observation optical system 40) based on the photographing condition selected. For example, the main controller 201 may perform control of the surgical microscope 10 in such a manner as to reapply the photographing condition selected. This makes it possible to reproduce the display mode of images when the value of the image parameter selected by the parameter selecting processor 2122 was applied in the past.

Regarding a photographing condition other than the controllable conditions of the surgical microscope 10 (e.g., the environmental condition, the subject's eye condition), for example, the parameter determining processor 2125 may execute processing based on the value of the image parameter selected and the photographing condition selected. This processing may be arithmetic processing executed based on a predetermined algorithm.

Some aspect examples are configured to prepare a correspondence (e.g., graph, table) between changes in the photographing condition (e.g., the environmental condition, the subject's eye condition) and values of image parameters. Then, the parameter determining processor 2125 may compare the current photographing condition with the selected photographing condition and determine a value of an image parameter based on the result of the comparison (e.g., the change amount of the photographing condition) and the correspondence prepared.

Instead of preparing the above correspondence, some aspects may introduce a system (artificial intelligent engine) that has learned a relationship between changes in the photographing condition (e.g., the environmental condition, the subject's eye condition) and values of image parameters using machine learning. This artificial intelligence engine includes, for example, a neural network constructed by machine learning with the photographing condition and the value of the image parameter as inputs and with a new value of an image parameter as an output. This artificial intelligence engine is trained, for example, in such a manner as to output an optimal image parameter values corresponding to a change in the photographing condition based on a given image parameter value. The parameter determining processor 2125 includes such an artificial intelligence engine, and inputs the value of the image parameter selected and the photographing condition selected into the artificial intelligence engine. Then, the value of the image parameter output from the artificial intelligence engine is used as a result obtained by the parameter determining processor 2125.

The image processor 211 (211A) may apply image processing using the value of the image parameter determined by the parameter determining processor 2125 to a moving image generated by the surgical microscope 10 (third moving image). As described above, the third moving image may be a moving image of any type, and may be the second moving image mentioned above, a moving image that has been acquired in another medical practice for the same subject, a moving image that has been acquired in a medical practice for another subject, or the like.

According to the present example, the value of the image parameter can be adjusted (corrected) in accordance with the difference in the photographing conditions. Therefore, it becomes possible to easily provide the third moving image that is represented in a manner similar to the previously achieved favorable display mode (e.g., color, brightness, contrast, etc.) regardless of differences in the photographing conditions.

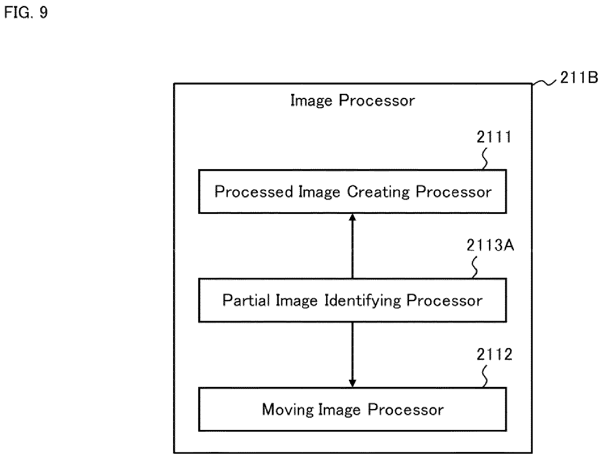

Next, an example shown in FIG. 9 will be described. The present example is configured to apply image processing only to a part of a still image (frame) included in a moving image. By doing so, the resources required for image processing is reduced and the time required for image processing is shortened, and also the quality of an image of a site of interest in observation of the subject's eye is optimized.

The image processor 211B shown in FIG. 9 creates a plurality of processed partial images by applying the first image processing to a part of a still image included in the first moving image generated by the surgical microscope 10. Here, the part of the still image is referred to as a partial image. In the present example, a plurality of processed partial images is treated as a plurality of processed images. The main controller 201 displays a plurality of images respectively including the created plurality of processed partial images on the display device 3. Each image displayed may be a corresponding processed partial image, or a wider area image that includes a corresponding processed partial image. Such a wide area image may be, for example, an image in which a partial image in a corresponding still image is replaced by a processed partial image. In this way, displaying the plurality of processed partial images allows the user to select a processed partial image that best depicts a site of interest.

The image processor 211B shown in FIG. 9 is an example of the image processor 211 shown in FIG. 4. The image processor 211B of the present example includes the processed image creating processor 2111 and the moving image processor 2112, as in the image processor 211A of FIG. 5. Unless otherwise mentioned, the processed image creating processor 2111 and the moving image processor 2112 of the present example may be the same as or similar to the processed image creating processor 2111 and the moving image processor 2112 in the example of FIG. 5, respectively.

The image processor 211B further includes the partial image identifying processor 2113A. The partial image identifying processor 2113A is configured to identify a partial image in a still image by applying segmentation for identifying an image of a specific site of the subject's eye to the still image included in the first moving image. In general, segmentation is image processing for identifying a partial region in a given image. Segmentation may include any known image processing technique, and some examples of which may include image processing such as edge detection and/or machine learning (e.g., deep learning) based segmentation.

The processed image creating processor 2111 of the present example is configured to apply the first image processing to the partial image identified by the partial image identifying processor 2113A. In other words, the application area of the first image processing by the processed image creating processor 2111 in the present example is limited to the partial image identified by the partial image identifying processor 2113A.

Further, the partial image identifying processor 2113A may be configured to apply the same or similar segmentation to the second moving image generated by the surgical microscope 10 after the selection of the processed image is performed by the user. More specifically, the partial image identifying processor 2113A may be configured to first apply segmentation for identifying an image of the same site of interest to each still image included in the second moving image (or, to each still image selected by thinning or like processing (the same applies hereinafter)).

In some aspects, the segmentation applied to a still image included in the second moving image may be performed in the same manner as the segmentation applied to a still image included in the first moving image. With this, an image of the same site of interest is identified from each still image included in the second moving image.

In some aspects, the ophthalmic observation apparatus 1 has a function of monitoring the movement of the subject's eye by analyzing a moving image generated by the surgical microscope 10. The partial image identifying processor 2113A stores the position of the subject's eye at the time of acquisition of a still image (to which segmentation has been applied) included in the first moving image. This position of the subject's eye is referred to as a reference position, and this still image is referred to as a reference still image. Further, the partial image identifying processor 2113A stores information indicating an area of a partial image in a still image included in the first moving image. This area is referred to as a partial image area. Upon beginning the generation of the second moving image, still images included in the second moving image (referred to as target still images) and the positions of the subject's eye at the times of the target still images are acquired (referred to as target positions) are input to the partial image identifying processor 2113A. The partial image identifying processor 2113A calculates the deviation of each target position with respect to the reference position and moves the partial image area by the calculated deviation, thereby identifying the area of the partial image in each target still image. According to the present example, tracking of the partial images in the second moving image, whose frames are acquired one after another, can be performed in real time even in the case where a target site is difficult to be segmented (e.g., a site depicted with a little difference in brightness or color from its surrounding area, a small site, etc.).

According to the present example configured to perform such processing, the partial image identifying processor 2113A can sequentially identify partial images of still images included in the second moving image by applying segmentation to the second moving image. Further, the image processor 210B (the moving image processor 2112) can sequentially apply the second image processing to the partial images identified from the still images included in the second moving image. As a result of this, the present example is capable of applying the value of the image parameter that has been applied to the processed partial image selected by the user, also to the second moving image. Thus, it becomes possible for the user to observe the second moving image in which the site of interest is appropriately depicted.

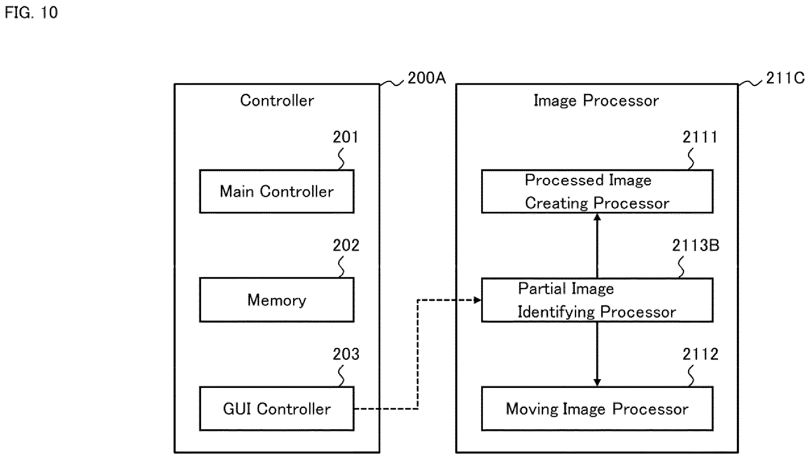

Next, an example shown in FIG. 10 will be described. As in the example in FIG. 9, the present example is configured to apply image processing only to a part of a still image (frame) included in a moving image, thereby reducing the resources required for image processing, shortening the time required for image processing, and optimizing the quality of an image of a site of interest in observation of the subject's eye.

The image processor 211C shown in FIG. 10 creates a plurality of processed partial images by applying the first image processing to a part (partial image) of a still image included in the first moving image generated by the surgical microscope 10. In the present example, a plurality of processed partial images is treated as a plurality of processed images. The main controller 201 displays a plurality of images respectively including the created plurality of processed partial images on the display device 3. Each image displayed may be a corresponding processed partial image, or a wider area image that includes a corresponding processed partial image. Such a wide area image may be, for example, an image in which a partial image in a corresponding still image is replaced by a processed partial image. In this way, by displaying the plurality of processed partial images, the user can select a processed partial image that best depicts the site of interest. While these features are the same as or similar to the example in FIG. 9, the present example differs from the example in FIG. 9 in the technique or method used to identify a partial image.

The image processor 211C shown in FIG. 10 is an example of the image processor 211 shown in FIG. 4. The image processor 211C of the present example includes the processed image creating processor 2111 and the moving image processor 2112, as in the image processor 211A of FIG. 5. Unless otherwise mentioned, the processed image creating processor 2111 and the moving image processor 2112 in the present example may be the same as or similar to the processed image creating processor 2111 and the moving image processor 2112 in the example of FIG. 5, respectively. The image processor 211C further includes the partial image identifying processor 2113B.

Further, the controller 200A shown in FIG. 10 is an example of the controller 200 in FIG. 3. The controller 200A of the present example includes the main controller 201 and the memory 202, as in the controller 200 of FIG. 3. Unless otherwise mentioned, the main controller 201 and the memory 202 in the present example may be the same as or similar to the main controller 201 and the memory 202 in the example of FIG. 3, respectively. The controller 200A further includes the graphical user interface (GUI) controller 203.

The main controller 201 displays the first moving image generated by the surgical microscope 10 (or a still image included in the first moving image (the same applies hereinafter)) on the display device 3. The GUI controller 203 displays a GUI used for designating a partial region in the first moving image on the display device 3. This GUI is, for example, an image of a figure having a shape and size similar to the site of interest. As an example of this, the site of interest is the pupil and the GUI is a circular or elliptical image. The user may change any of the position, size, and shape of the GUI by using, for example, the operation device 2. With this, for example, the user may perform an operation of matching the GUI with the outer edge of a pupil image in the first moving image. After such adjustment of the GUI with respect to the image of the site of interest is done, the GUI controller 203 may perform tracking of the GUI in accordance with the movement of the subject's eye in the first moving image. The partial image identifying processor 2113B identifies an area specified by the GUI (e.g., an area surrounded or defined by the GUI (and a certain area around it)) as a partial region.

The processed image creating processor 2111 of the present example is configured to apply the first image processing to the partial image identified by the partial image identifying processor 2113B. In other words, the application area of the first image processing by the processed image creating processor 2111 in the present example is limited to the partial image identified by the partial image identifying processor 2113B.

Further, the partial image identifying processor 2113B may identify a partial image in the second moving image generated by the surgical microscope 10 after the selection of the processed image has been performed by the user, for example, in the same manner as the tracking described above.

According to the present example configured to perform such processing, the partial image identifying processor 2113B may sequentially identify partial images corresponding to the site of interest in still images included in the second moving image by applying segmentation to the second moving image. Further, the image processor 210C (the moving image processor 2112) may sequentially apply the second image processing to the partial images identified from the still images included in the second moving image. As a result of this, the value of the image parameter that has been applied to the processed partial image selected by the user can also be applied to the second moving image, making it possible for the user to observe the second moving image in which the site of interest is appropriately depicted. In addition, the present example is capable of setting an area designated by the user to be a partial image. Therefore, it becomes possible to represent the area according to the user's preference in a manner according to the user's preference.

### < Operation/usage mode>

Some examples of the operation and the usage mode of the ophthalmic observation apparatus 1 will be described.

### < First example >

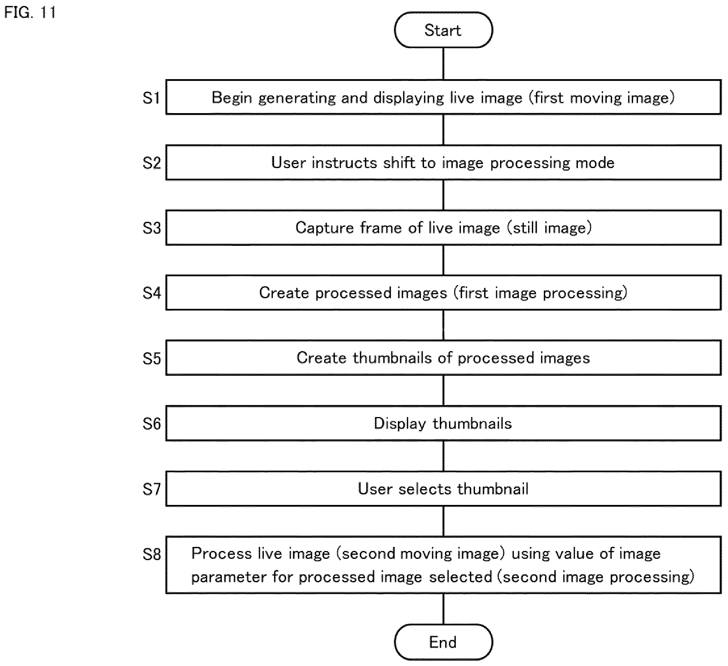

The first example of the operation and usage mode of the ophthalmic observation apparatus 1 will be described with reference to FIG. 11 to 12E. It should be noted that any items or matters of the above-described processes, operations, and usage modes may be combined with the present example.

### (S1: Begin generating and displaying a live image)

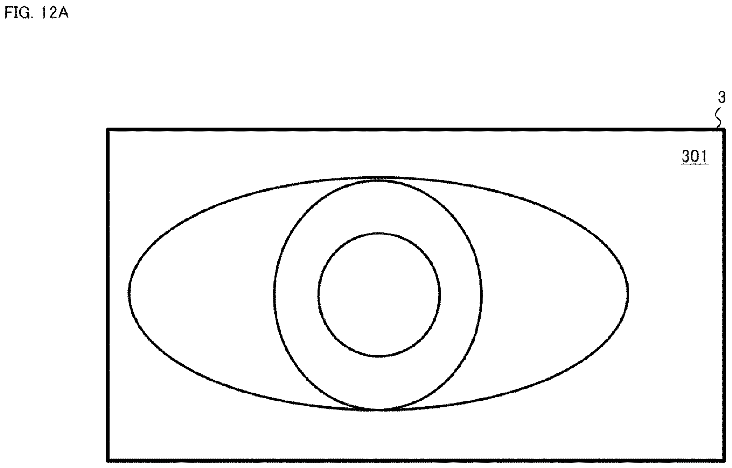

First, the user instructs the ophthalmic observation apparatus 1 to start the generation and display of a live image of the subject's eye by performing a predetermined operation using the operation device 2. Specifically, the surgical microscope 10 generates digital image data (video) of the subject's eye by the image sensors 62L and 62R while illuminating the subject's eye by the illumination optical system 30. The video generated (the live image 301) is displayed on the display device 3 in real time (see FIG. 12A). In other words, the video acquired by the surgical microscope 10 is displayed as a live motion picture (live moving image, live image) on the display device 3. The user can perform surgery while observing the live image. This live image corresponds to the first moving image described above.

### (S2: Shift to image processing mode)

Next, the user performs an instruction to switch the operation mode of the ophthalmic observation apparatus 1 to the image processing mode by performing a predetermined operation using the operation device 2. The image processing mode is an operation mode for applying processing on the live image displayed by the ophthalmic observation apparatus 1.

### (S3: Capture frame)

After the operation mode has changed to the image processing mode, the ophthalmic observation apparatus 1 captures a frame(s) (still image(s)) of the live image.

The number of frames to be captured is freely selected. In the case where one frame is captured, this frame is subjected to subsequent processing. In the case where two or more frames are captured, the ophthalmic observation apparatus 1 (e.g., the data processor 210) may select one frame from these captured frames or generate one still image from these captured frames. In some examples, the ophthalmic observation apparatus 1 may be configured to calculate image quality evaluation values of the two or more captured frames and compare the image quality evaluation values to select one frame. In some other examples, the ophthalmic observation apparatus 1 may be configured to display two or more captured frames on the display device 3 and select one frame designated by the user using the operation device 2. In some yet other examples, the ophthalmic observation apparatus 1 may be configured create one frame by synthesizing (composing) two or more captured frames by image processing.

### (S4: First image processing)

The data processor 210 (the image processor 211, the processed image creating processor 2111) applies the first image processing with a plurality of different values of each of one or more image parameters, to the frame(s) captured in the step S3. As a result of this, a plurality of processed images based on the captured frame(s) is created.

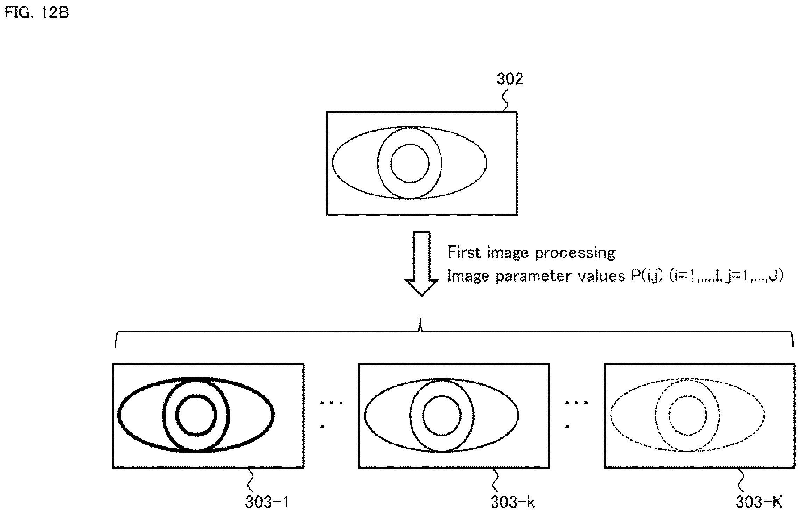

In the example shown in FIG. 12B, each of the K number of pieces of image processing included in the first image processing is applied to the frame (still image) 302 that has been captured from the live image 301. The I number of image parameters (the I number of types of image parameters) are used for the K number of pieces of image processing (here, the "I" is an integer equal to or greater than 1). Further, the J number of values are prepared for each of the image parameters (i.e., for each i-th image parameter; i = 1, ..., I) (here, the "J" is an integer equal to or greater than 2). Note that the number of prepared values, J, may be equal or different among all image parameters. In the former case, K = I × J. In the latter case, K = Σ [J(i)]. Here, J(i) indicates the number of values for the i-th image parameter, and Σ is the summation over all i. The j-th value of the i-th image parameter is denoted by P(i, j). Applying the first image processing designed in this way generates the plurality of processed images 303-k. Here, k = 1, ..., K, where K is an integer equal to or greater than 2.

Here, for each of the plurality of processed images, the main controller 201 or the data processor 210 associates, with that processed image, the value(s) (one or more values) of the image parameter(s) used to create that processed image, and records the value(s) in association with that processed image. For example, in the example of FIG. 12B, for each processed image 303-k, one or more values P (i, j) of one or more image parameters used to create this processed image 303-k are associated with this processed image 303-k. Information that indicates such association between processed images and values of image parameters is referred to as association information herein.

### (S5: Create thumbnails of processed images)

Next, the data processor 210 (the image processor 211) creates a thumbnail of each of the plurality of processed images created in the step S4.

### (S6: Display thumbnails)

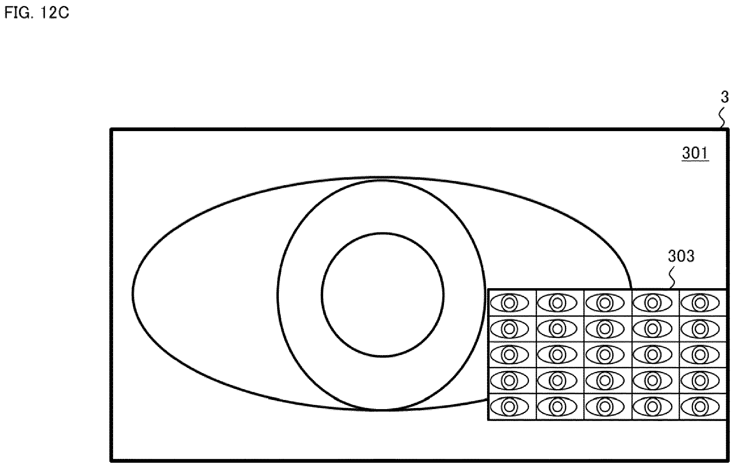

Next, the main controller 201 displays the plurality of thumbnails created in the step S5 on the display device 3. In the present example, as shown in FIG. 12C, the plurality of thumbnails (thumbnail group) 303 arranged in a predetermined array is displayed over the live image 301.

The number of thumbnails displayed at one time (simultaneously) is freely selected. For example, all the thumbnails created in the step S5 may be presented at one time, or one or more (not all) of all the thumbnails created in the step S5 may be presented at one time (e.g., a predetermined number of thumbnails from all the thumbnails created in the step S5 may be presented at one time). Further, the number of thumbnails presented at one time may be changed. In the case where only a part (one or more (not all)) of all the thumbnails created in the step S5 is presented at one time, the user may perform an operation for changing thumbnails presented. This operation may be, for example, page switching or scrolling.

### (S7: Select thumbnail)

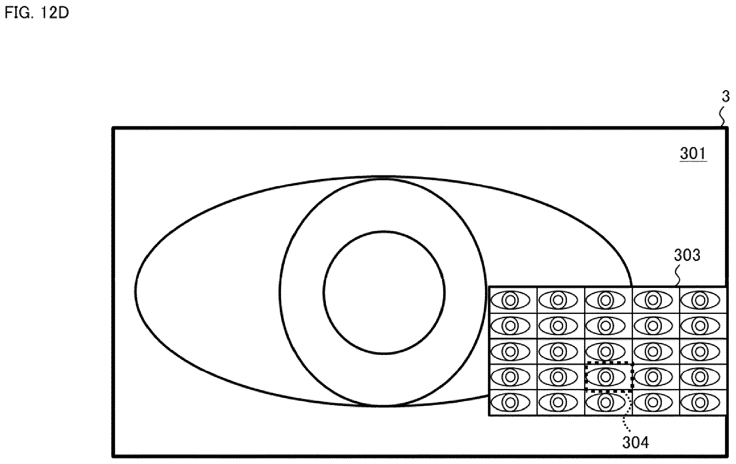

The user compares the plurality of thumbnails displayed in the step S6 and selects a desired thumbnail. This selection operation is performed using the instruction receiving unit described above such as the foot switch included in the operation device 2. In the example shown in FIG. 12D, one thumbnail 304 is selected from the plurality of thumbnails 303.

### (S8: Second image processing)

The main controller 201 or the data processor 210 identifies the value(s) of the image parameter(s) associated with the processed image corresponding to the thumbnail selected in the step S7, by referring to the association information described above.

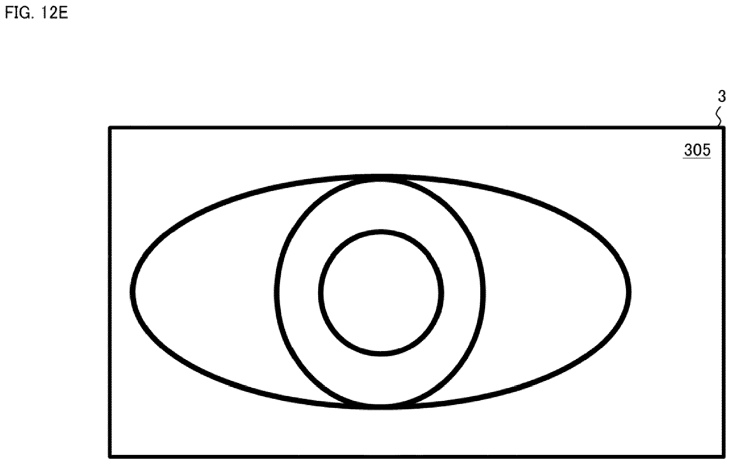

Furthermore, the data processor 210 (the image processor 211, the moving image processor 2112) uses the identified value(s) of the image parameter(s) to execute processing of the live image being generated by the surgical microscope 10 in real time (second image processing). The live image processed in real time is displayed in real time on the display device 3 by the main controller 201 (End). Note that the live image generated and displayed at this stage corresponds to the second moving image mentioned above (the live image 305 in FIG. 12E).

The user may perform surgery while observing the live image 305 obtained by applying the same image processing as that applied to the processed image selected by the user.

The ophthalmic observation apparatus 1 may be configured to read out a value(s) of an image parameter(s) applied in the past and use the value(s) again. In this case, the ophthalmic observation apparatus 1 may be configured to select and read out a value(s) of an image parameter(s) that was (were) used by this user in the past, and use the value(s) again. Alternatively, the ophthalmic observation apparatus 1 may be configured to select and read out a value(s) of an image parameter(s) that is (are) suitable for an attribute (e.g., the type of surgery) of the medical practice currently being performed, and use the value(s) again. In some other examples, the ophthalmic observation apparatus 1 may be configured to select and read out a value(s) of an image parameter(s) that is (are) suitable for a phase of the medical practice currently being performed, and use the value(s) again. In some yet other examples, the ophthalmic observation apparatus 1 may be configured to select and read out a plurality of values of an image parameter(s) that are respectively suitable for a plurality of phases of the medical practice currently being performed, and reuse the plurality of values sequentially.

In the case of performing anterior segment surgery, for example, one or more of a color tone parameter, a brightness parameter, a contrast parameter, a gain parameter, and other parameters may be used as an image parameter(s). This allows the user, for example, in cataract surgery, to observe a site such as the cornea, the iris, the pupil, or the crystalline lens in a preferred display mode by the user, and to clearly grasp an incision, a side port, an anterior capsule incision site in CCC, the state of phacoemulsification and the state of aspiration of the crystalline lens, the position and the orientation of an IOL, and other states and conditions. In addition, such a suitable image display mode can be achieved easily and quickly.

In the case of performing vitreous body surgery or posterior segment surgery, for example, one or more of a gamma parameter, a gain parameter, a color temperature parameter, a white balance parameter, an RGB balance parameter, and other parameters may be used as an image parameter(s). This allows a floating object (floater) in the vitreous body, an opacity, a proliferative membrane, or the like to be highlighted, thereby improving the work efficiency of surgery and avoiding or reducing surgical errors.

### < Second example >

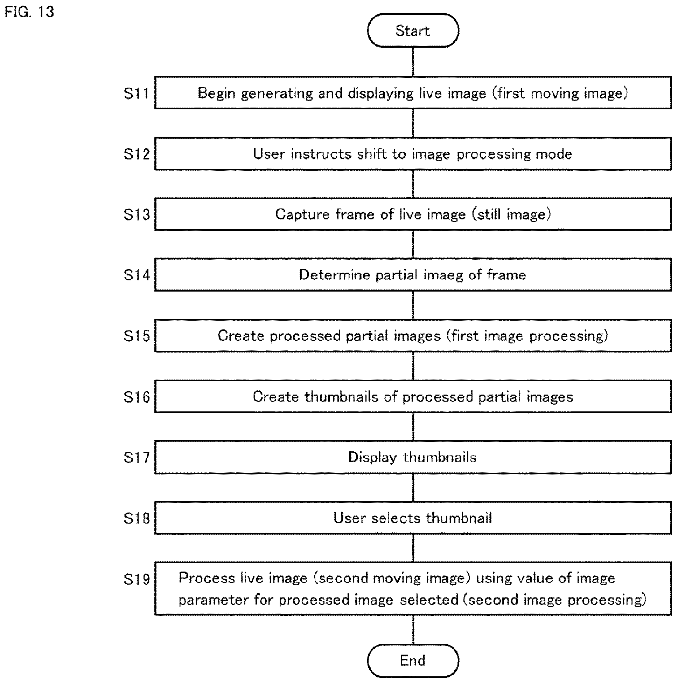

The second example of the operation and usage mode of the ophthalmic observation apparatus 1 will be described with reference to FIG. 13 to FIG. 14G. Note that any items or matters of the above-mentioned processes, operations, and usage modes may be combined with the present example.

### (S11: Begin generating and displaying a live image)

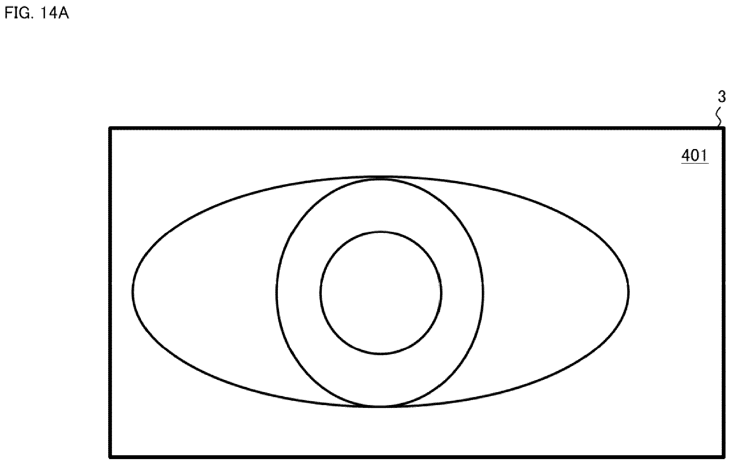

First, the user instructs the ophthalmic observation apparatus 1 to start the generation and display of a live image of the subject's eye by performing a predetermined operation using the operation device 2. Specifically, the surgical microscope 10 generates digital image data (video) of the subject's eye by the image sensors 62L and 62R while illuminating the subject's eye by the illumination optical system 30. The video generated (the live image 401) is displayed on the display device 3 in real time (see FIG. 14A). In other words, the video acquired by the surgical microscope 10 is displayed as a live motion picture (live moving image, live image) on the display device 3. The user can perform surgery while observing the live image. This live image corresponds to the first moving image described above.

### (S12: Shift to image processing mode)

Next, the user performs an instruction to switch the operation mode of the ophthalmic observation apparatus 1 to the image processing mode by performing a predetermined operation using the operation device 2. The image processing mode is an operation mode for applying processing on the live image displayed by the ophthalmic observation apparatus 1.

### (S13: Capture frame)

After the operation mode has changed to the image processing mode, the ophthalmic observation apparatus 1 captures a frame(s) (still image(s)) of the live image.

The number of frames to be captured is freely selected. In the case where one frame is captured, this frame is subjected to subsequent processing. In the case where two or more frames are captured, the ophthalmic observation apparatus 1 (e.g., the data processor 210) may select one frame from these captured frames or generate one still image from these captured frames. In some examples, the ophthalmic observation apparatus 1 may be configured to calculate image quality evaluation values of the two or more captured frames and compare the image quality evaluation values to select one frame. In some other examples, the ophthalmic observation apparatus 1 may be configured to display two or more captured frames on the display device 3 and select one frame designated by the user using the operation device 2. In some yet other examples, the ophthalmic observation apparatus 1 may be configured create one frame by synthesizing (composing) two or more captured frames by image processing.

### (S14: Determine partial image)

Next, the ophthalmic observation apparatus 1 (the image processor 211, the partial image identifying processor 2113A or 2113B) determines a partial image of the frame captured in the step S13.

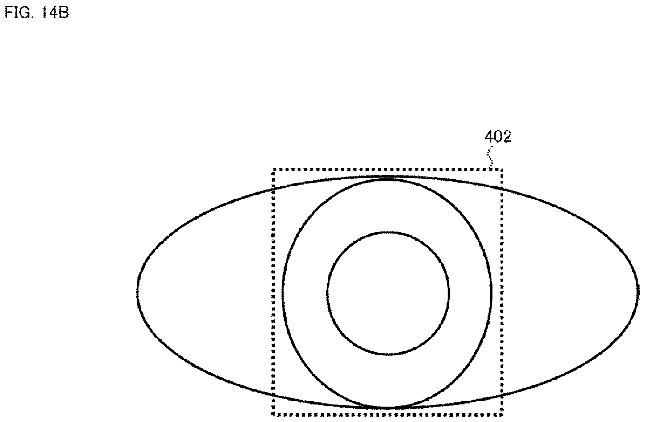

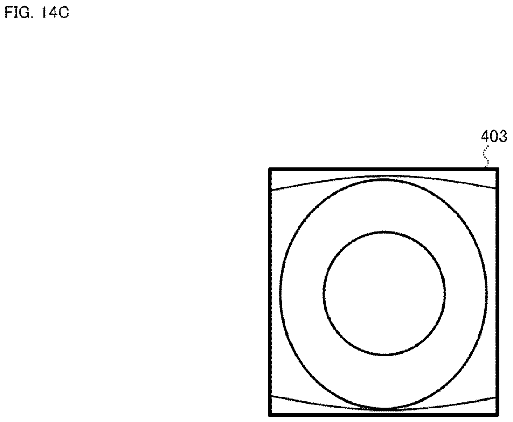

The processing of the present step may be performed automatically or manually. In some automatic cases, the partial image identifying processor 2113A of FIG. 9 may apply segmentation to a frame captured in the step S13 to identify a partial image of the frame (the image region surrounded by the frame 402 shown in FIG. 14B). In some manual cases, for example, the main controller 201 may display a frame captured in the step S13 on the display device 3, and the GUI controller 203 may display a predetermined GUI on this frame. Then, the user may operate the GUI to designate a partial region of the frame. Then, the partial image identifying processor 2113B may identify an area designated using the GUI (e.g., the area surrounded by the GUI (and a certain area around this area)) as a partial image (the image region surrounded by the frame 402 shown in FIG. 14B). As a result, for example, the partial image 403 shown in FIG. 14C is obtained.

### (S15: First image processing)

The data processor 210 (the image processor 211, the processed image creating processor 2111) applies the first image processing with a plurality of different values of each of one or more image parameters, to the partial image(s) obtained by the step S14. As a result of this, a plurality of processed images based on the partial image(s) is created.

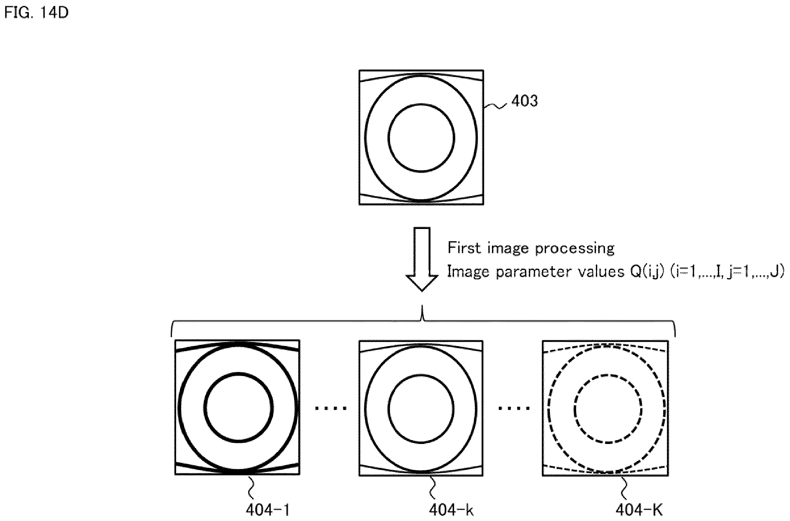

In the example shown in FIG. 14D, each of the K number of pieces of image processing included in the first image processing is applied to the partial image 403. The I number of image parameters (the I number of types of image parameters) are used for the K number of pieces of image processing (here, the "I" is an integer equal to or greater than 1). Further, the J number of values are prepared for each of the image parameters (i.e., for each i-th image parameter; i = 1, ..., I) (here, the "J" is an integer equal to or greater than 2). Note that the number of prepared values, J, may be equal or different among all image parameters. In the former case, K = I × J. In the latter case, K = Σ [J(i)]. Here, J(i) indicates the number of values for the i-th image parameter, and Σ is the summation over all i. The j-th value of the i-th image parameter is denoted by Q(i, j). Applying the first image processing designed in this way generates the plurality of processed partial images 404-k. Here, k = 1, ..., K, where K is an integer equal to or greater than 2.

Here, for each of the plurality of processed partial images (or the plurality of processed images), the main controller 201 or the data processor 210 associates, with that processed partial image, the value(s) (one or more values) of the image parameter(s) used to create that processed partial image, and records the value(s) in association with that processed partial image. For example, in the example of FIG. 14D, for each processed partial image 404-k (or a processed image corresponding to this processed partial image), one or more values Q(i, j) of one or more image parameters used to create this processed partial image 404-k are associated with this processed partial image 404-k. Information that indicates such association between processed partial images and values of image parameters is referred to as association information herein.

### (S16: Create thumbnails of processed partial images)

Next, the data processor 210 (the image processor 211) creates a thumbnail of each of the plurality of processed partial images created in the step S15.

### (S17: Display thumbnails)

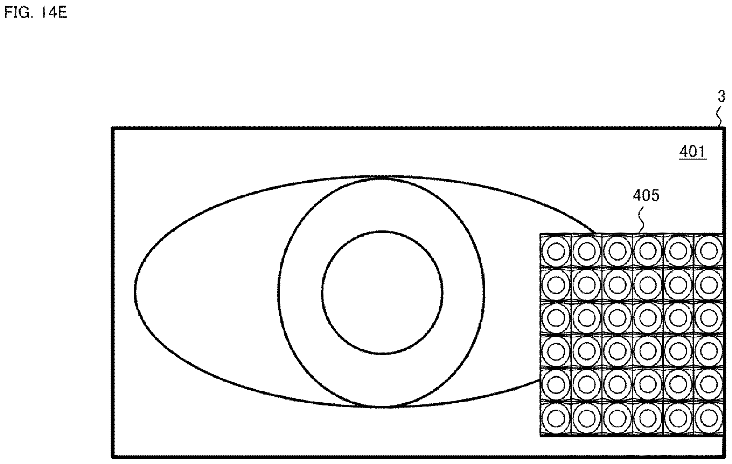

Next, the main controller 201 displays the plurality of thumbnails created in the step S16 on the display device 3. In the present example, as shown in FIG. 14E, the plurality of thumbnails (thumbnail group) 405 arranged in a predetermined array is displayed on the live image 401.

The number of thumbnails displayed at one time (simultaneously) is freely selected. For example, all the thumbnails created in the step S16 may be presented at one time, or one or more (not all) of all the thumbnails created in the step S16 may be presented at one time (e.g., a predetermined number of thumbnails from all the thumbnails created in the step S16 may be presented at one time). Further, the number of thumbnails presented at one time may be changed. In the case where only a part (one or more (not all)) of all the thumbnails created in the step S16 is presented at one time, the user may perform an operation for changing thumbnails presented. This operation may be, for example, page switching or scrolling.

### (S18: Select a thumbnail)

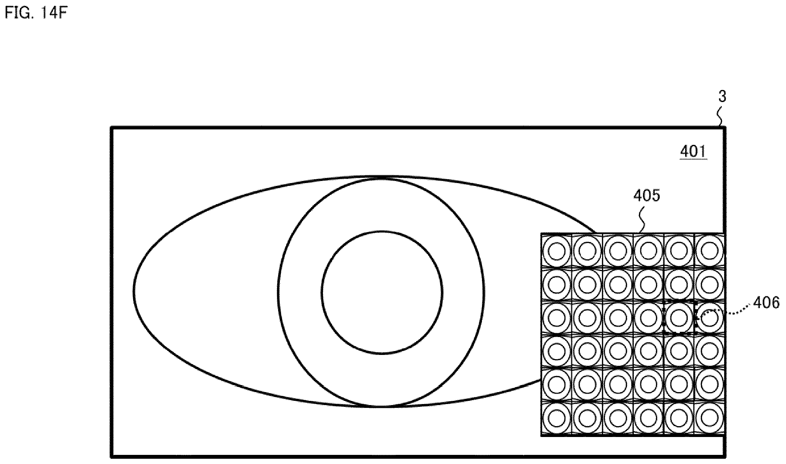

The user compares the plurality of thumbnails displayed in the step S17 and selects a desired thumbnail. This selection operation is performed using the instruction receiving unit described above such as the foot switch included in the operation device 2. In the example shown in FIG. 14F, one thumbnail 406 is selected from the plurality of thumbnails 405.

### (S19: Second image processing)

The main controller 201 or the data processor 210 identifies the value(s) of the image parameter(s) associated with the processed partial image corresponding to the thumbnail selected in the step S18 by referring to the association information described above.

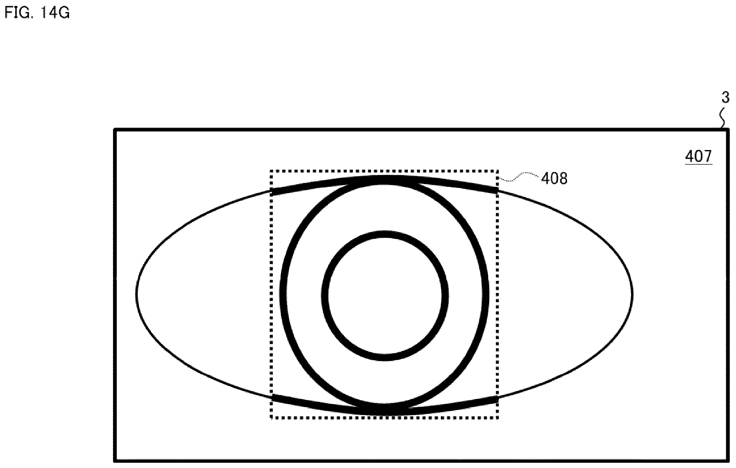

Furthermore, the data processor 210 (the image processor 211, the moving image processor 2112) uses the identified value(s) of the image parameter(s) to execute processing of the live image being generated by the surgical microscope 10 in real time (second image processing). The area to which the second image processing is applied may be only a region of the live image (a frame thereof) corresponding to the frame 402 of FIG. 14B, or may be the entire live image (an entire frame thereof). In the former case, for example, regions of the live image corresponding to the frame 402 are sequentially identified by the tracking described above. The live image processed in real time is displayed in real time on the display device 3 by the main controller 201 (End). Note that the live image generated and displayed at this stage corresponds to the second moving image mentioned above. The live image 407 of FIG. 14G is an example of a live image displayed in this way. In the live image 407, the second image processing is applied only to the region corresponding to the frame 402 (the region surrounded by the frame 408).

The user may perform surgery while observing the live image 407 obtained by applying the same image processing as that applied to the processed image selected by the user.

The ophthalmic observation apparatus 1 may be configured to read out a value(s) of an image parameter(s) applied in the past and use the value(s) again. In this case, the ophthalmic observation apparatus 1 may be configured to select and read out a value(s) of an image parameter(s) that was (were) used by this user in the past, and use the value(s) again. Alternatively, the ophthalmic observation apparatus 1 may be configured to select and read out a value(s) of an image parameter(s) that is (are) suitable for an attribute (e.g., the type of surgery) of the medical practice currently being performed, and use the value(s) again. In some other examples, the ophthalmic observation apparatus 1 may be configured to select and read out a value(s) of an image parameter(s) that is (are) suitable for a phase of the medical practice currently being performed, and use the value(s) again. In some yet other examples, the ophthalmic observation apparatus 1 may be configured to select and read out a plurality of values of an image parameter(s) that are respectively suitable for a plurality of phases of the medical practice currently being performed, and reuse the plurality of values sequentially.

In the case of performing anterior segment surgery, for example, one or more of a color tone parameter, a brightness parameter, a contrast parameter, a gain parameter, and other parameters may be used as an image parameter(s). This allows the user, for example, in cataract surgery, to observe a site such as the iris, the pupil, or the crystalline lens in a preferred display mode by the user, and to clearly grasp an incision, a side port, an anterior capsule incision site in CCC, the state of phacoemulsification and the state of aspiration of the crystalline lens, the position and the orientation of an IOL, and other states and conditions. In addition, such a suitable image display mode can be achieved easily and quickly.

In the case of performing vitreous body surgery or posterior segment surgery, for example, one or more of a gamma parameter, a gain parameter, a color temperature parameter, a white balance parameter, an RGB balance parameter, and other parameters may be used as an image parameter(s). This allows a floating object (floater) in the vitreous body, an opacity, a proliferative membrane, or the like to be highlighted, thereby improving the work efficiency of surgery and avoiding or reducing surgical errors.

### < Modification examples >

Some modification examples of the embodiment examples described above will be described.

In the examples shown in FIG. 12C, FIG.12D, FIG. 14E, and FIG.14F, a part of an image of the subject's eye is hidden by a plurality of thumbnails (processed images or processed partial images). In order to avoid such a situation, a plurality of thumbnails may be displayed within a region where an image of the subject's eye is not displayed. Examples of processing employable for achieving this may include any of the following processes: a process of reducing the display size of a plurality of thumbnails; a process of reducing the display size of each thumbnail; a process of changing the arrangement of a plurality of thumbnails; and a process of reducing the number of thumbnails simultaneously displayed. Some examples may detect the area of an image of the subject's eye and display a plurality of thumbnails in an area other than the detected area.

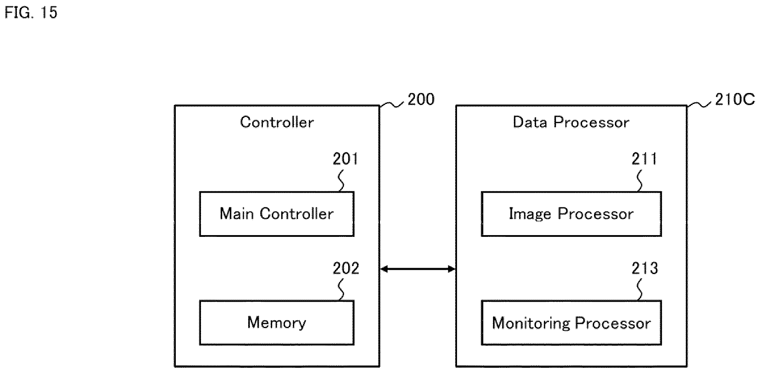

FIG. 15 shows an example of a configuration that may be employed for achieving such an operation. The data processor 210C of FIG. 15 is an example of the data processor 210 of FIG. 3. The data processor 210C of the present example includes the monitoring processor 213 in addition to the image processor 211. The image processor 211 may have the same configuration and execute the same operation as those of the embodiment examples described above.

The monitoring processor 213 is configured to perform data processing for monitoring the movement of the subject's eye (monitoring processor). In some examples, the monitoring processor 213 detects a feature point of the subject's eye by analyzing a frame of a moving image generated by the surgical microscope 10. The feature point may be any landmark, such as the pupil (its center, outer edge, etc.), the corneal ring (the outer edge of the iris), the corner angle, the optic nerve head, the macula, a blood vessel, or other sites or tissues. The monitoring processor 213 sequentially analyzes frames that are sequentially generated as a real-time moving image (first moving image), thereby detecting the movement of a feature point in the moving image (the change with time in the position of a feature point in the moving image) in real time.

The main controller 201 may change the display state of a plurality of processed images (a plurality of processed partial images, a plurality of thumbnails) displayed together with the real-time moving image based on an output from the monitoring processor 213. This display state change control is performed, for example, in such a manner as to display the plurality of processed images in an area where the image of the subject's eye is not displayed. For example, the main controller 201 may perform any of the following controls: control to reduce the display size of the plurality of processed images; control to reduce the display size of each processed image; control to change the arrangement of the plurality of processed images; control to reduce the number of the plurality of processed images displayed at a time; and control to stop displaying the plurality of processed images (control not to display the plurality of processed images).

The present modification example thus configured is capable of (dynamically) changing the display state of the plurality of processed images in accordance with the movement of the subject's eye. With this, the plurality of processed images does not interfere with the observation of the image of the subject's eye. Note that the configuration for detecting the movement of the subject's eye (the monitoring processor) is not limited to the configuration and processing described above, and may be freely designed and configured.

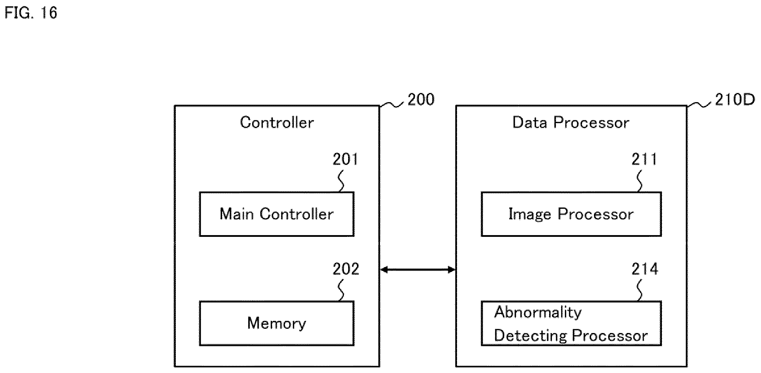

FIG. 16 shows another modification example. While the modification example of FIG. 15 focuses on the movement of the subject's eye, the present modification example focuses on the occurrence of an abnormality in the subject's eye.

The data processor 210D in FIG. 16 is an example of the data processor 210 in FIG. 3. The data processor 210D of the present example includes the abnormality detecting processor 214 in addition to the image processor 211. The image processor 211 may have the same configuration and execute the same operation as those of the embodiment examples described above.

The abnormality detecting processor 214 is configured to execute data processing for detecting an abnormality in the subject's eye. The type of the abnormality to be detected may be freely selected, and may be bleeding, wound, or other abnormalities, for example. The abnormality detecting processor 214 detects an abnormality by, for example, analyzing a frame of a moving image generated by the surgical microscope 10. For example, bleeding may be detected based on changes in colors in an image (such as an increase in red regions).

In some aspects, the abnormality detecting processor 214 may include a system (artificial intelligence engine) constructed by machine learning using a training dataset that includes surgical images or the like. This artificial intelligence engine includes, for example, a neural network (typically, a convolutional neural network) constructed by machine learning with a surgical image as an input and a probability of the occurrence of a predetermined abnormality as an output. The abnormality detecting processor 214 of the present example sequentially inputs frames of a real-time moving image (first moving image) generated by the surgical microscope 10 to the artificial intelligence engine. The artificial intelligence engine sequentially outputs the probabilities of the occurrences of the abnormality based on the frames input. The abnormality detecting processor 214 executes abnormality detection on the basis of the probabilities of the occurrences of the abnormality sequentially output from the artificial intelligence engine. In some examples, the abnormality detecting processor 214 may determine that the abnormality is detected if the abnormality occurrence probabilities that are sequentially output exceed a preset threshold value. In some other examples, the abnormality detecting processor 214 may perform abnormality detection based on a change in the abnormality occurrence probabilities that are sequentially output (e.g., based on the rate of the change, the amount of the change, or the like).

The main controller 201 may change the display state of a plurality of processed images (a plurality of processed partial images, a plurality of thumbnails) displayed together with a real-time moving image, based on an output from the abnormality detecting processor 214. This display state change control may be performed, for example, in such a manner as to display the plurality of processed images in an area where the image of the subject's eye is not displayed, or in such a manner as to display the plurality of processed images in an area other than the area where an abnormality is detected. In some examples, the main controller 201 may perform the following controls: control to reduce the display size of the plurality of processed images; control to reduce the display size of each processed image; control to change the arrangement of the plurality of processed images; control to reduce the number of the plurality of processed images displayed at a time; and control to stop displaying the plurality of processed images (control not to display the plurality of processed images).

The present modification example thus configured is capable of (dynamically) changing the display state of the plurality of processed images in response to an event that the occurrence of an abnormality in the subject's eye is detected. With this, the plurality of processed images does not interfere with the observation of the image of the subject's eye (in particular, observation of the location where the abnormality has occurred). Note that the configuration for detecting the abnormality of the subject's eye (the abnormality detecting processor) is not limited to the configuration and processing described above, and may be freely designed and configured.

The ophthalmic observation apparatus 1 of the above embodiment examples is configured to generate and display a plurality of processed images by applying the first image processing to a still image(s) (frame(s)) included in the first moving image. In contrast, some aspect examples may be configured to generate and display a plurality of processed images (a plurality of processed moving images) by sequentially applying the first image processing to each still image (each frame) included in the first moving image. Such aspect examples may display the plurality of processed moving images side by side or sequentially. Further, such aspect examples may display a still image(s) (processed still image(s), processed frame(s)) included in processed images for one or more among the plurality of processed moving images. The present aspect is effective in the case where a display mode of a moving subject's eye is desired to be optimized, or in the case where a display mode of a moving object (floating object, opacity, proliferative membrane, liquid flow, blood flow, or other moving objects) is desired to be optimized. On the other hand, executing such processing in real time requires a large quantity of resources exceeding those for the ophthalmic observation apparatus 1 in the embodiment examples described above. Therefore, in order to reduce the processing load for handling a processed moving image(s), any method or technique of reducing processing load may be applied such as any of the following processes: a process of reducing the number of processed moving images to be generated; a process of reducing the number of processed moving images (thumbnails) to be processed; a process of reducing the number of processed moving images (thumbnails) to be displayed; and a process of thinning out frames.

### < Ophthalmic image processing apparatus >

An ophthalmic image processing apparatus not claimed in this patent application is described below. The ophthalmic image processing apparatus is configured to process an image of a subject's eye. Any items or matters (functions, configurations, processing, operations, usage modes, etc.) relating to the ophthalmic observation apparatus 1 of the above embodiment examples may be combined with the example of the ophthalmic image processing apparatus described below.

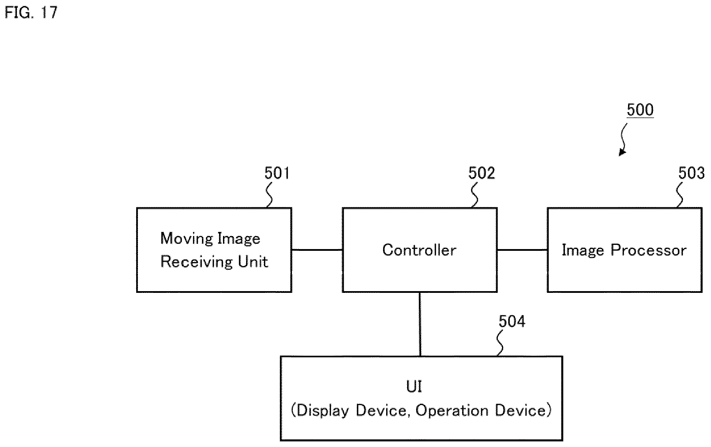

FIG. 17 shows an example of the configuration of the ophthalmic image processing apparatus of the present example. Among the elements of the ophthalmic image processing apparatus 500 shown in FIG. 17, the elements other than the moving image receiving unit 501 may be configured in the same manner as the corresponding elements in the ophthalmic observation apparatus 1 according to the embodiment examples described above, and detailed descriptions of these elements will be omitted unless otherwise mentioned.

The controller 502 of the ophthalmic image processing apparatus 500 corresponds to the controller 200 of the ophthalmic observation apparatus 1 of the above embodiment examples. The image processor 503 of the ophthalmic image processing apparatus 500 corresponds to the data processor 210 (the image processor 211) of the ophthalmic observation apparatus 1 of the above embodiment examples. The user interface (UI) 504 of the ophthalmic image processing apparatus 500 corresponds to the operation device 2 and the display device 3 of the ophthalmic observation apparatus 1 of the above embodiment examples.

The moving image receiving unit 501 is configured to receive a moving image of the subject's eye. The moving image receiving unit 501 receives a moving image directly or indirectly from the surgical microscope 10. In some examples, the moving image receiving unit 501 is connected to the surgical microscope 10 by a communication line or a cable. In some other examples, the moving image receiving unit 501 is connected to a device (buffer), in which a moving image generated by the surgical microscope 10 is (temporarily) stored, by a communication line or a cable.

The moving image receiving unit 501 receives the first moving image of the above embodiment examples. The controller 502 sends the first moving image received by the moving image receiving unit 501 to the image processor 503.

By executing the processing described in the above embodiment examples, the image processor 503 applies the first image processing using a plurality of different values of a predetermined image parameter to a still image included in the first moving image, thereby creating a plurality of processed images.

The controller 502 (display controller) displays the plurality of processed images created by the image processor 503 on the UI 504 (first display device).

In addition to such a series of operations, the ophthalmic image processing apparatus 500 may perform the following processing in the same manner as the ophthalmic observation apparatus 1 of the above embodiment examples. The user may give an instruction for selecting at least one processed image from among the plurality of processed images displayed on the UI 504. This instruction may be performed in the same manner as in the case of the ophthalmic observation apparatus 1 of the above embodiment examples, and is performed using, for example, the UI 504 (instruction receiving unit).

The image processor 503 applies the second image processing to a moving image of the subject's eye (second moving image). Here, the second image processing is image processing on the basis of at least one value of an image parameter corresponding to at least one processed image designated by the user, and the second moving image is a moving image of the subject's eye received by the moving image receiving unit 501 after selection based on the user's instruction is performed. This processing (second image processing) is executed in the same manner as in the case of the ophthalmic observation apparatus 1 of the above embodiment examples.

The controller 502 (display controller) displays the second moving image to which the second image processing has been applied, on the UI 504 (second display device).

The ophthalmic image processing apparatus 500 thus configured is capable of achieving the same actions and effects as those of the ophthalmic observation apparatus 1 of the above embodiment examples. In addition, by combining any of the matters and items (functions, configurations, processing, operations, usage modes, etc.) relating to the ophthalmic observation apparatus 1 of the above embodiment examples with the ophthalmic image processing apparatus 500, the resulting ophthalmic image processing apparatus 500 becomes capable of achieving the actions and effects corresponding to the combined matters and/or items.

### < Ophthalmic image processing method >

Some embodiment examples (e.g., the ophthalmic observation apparatus 1 or the ophthalmic image processing apparatus 500 described above) provide a method of processing an ophthalmic image (an image of a subject's eye). It is possible to combine any items or matters relating to the ophthalmic observation apparatus 1 of the above embodiment examples with the following example of an ophthalmic image processing method. In addition, it is possible to combine any items or matters relating to the ophthalmic image processing apparatus 500 of the above embodiment example with the following example of an ophthalmic image processing method.

The ophthalmic image processing method of some aspect examples first receives the first moving image of a subject's eye. Next, the method creates a plurality of processed images by applying the first image processing using a plurality of different values of a predetermined image parameter to a still image included in the first moving image. Next, the method displays the plurality of processed images created. Subsequently, the method receives an instruction for selecting at least one processed image from among the plurality of processed images displayed. The method receives the second moving image of the subject's eye after selection of the at least one processed image is performed based on the instruction. Following this, the method applies the second image processing based on at least one value of an image parameter corresponding to the at least one selected processed image to the second moving image. Then, the method displays the second moving image to which the second image processing has been applied. Such a series of steps is described as the operation and usage mode of the ophthalmic observation apparatus 1 of the above embodiment examples (refer to FIG. 11, FIG. 12A to FIG. 12E, FIG. 13, FIG. 14A to 14G, etc.).

The ophthalmic image processing method thus configured is capable of achieving the same actions and effects as those of the ophthalmic observation apparatus 1 and the ophthalmic image processing apparatus 500 of the above embodiment examples. In addition, by combining any of the matters and items relating to the ophthalmic observation apparatus 1 or the ophthalmic image processing apparatus 500 of the above embodiment examples with the ophthalmic image processing method, the resulting ophthalmic image processing method becomes capable of achieving the actions and effects corresponding to the combined matters and/or items.

### < Program >

Some embodiment examples provide a program - not claimed in this patent application - causing a computer to execute the ophthalmic image processing method described above. It is possible to combine any of the matters and items relating to the ophthalmic observation apparatus 1 or the ophthalmic image processing apparatus 500 of the above embodiment examples with such a program.

The program thus configured is capable of achieving the same actions and effects as those of the ophthalmic observation apparatus 1 and the ophthalmic image processing apparatus 500 of the above embodiment examples. In addition, by combining any of the matters and items relating to the ophthalmic observation apparatus 1 or the ophthalmic image processing apparatus 500 of the above embodiment examples with the program, the resulting program is capable of achieving the actions and effects corresponding to the combined matters and/or items.

### < Recording medium >

According to the invention, a computer-readable non-transitory recording medium storing the program described above is provided. It is possible to combine any of the matters and items relating to the ophthalmic observation apparatus 1 or the ophthalmic image processing apparatus 500 of the above embodiment examples with such a recording medium. The non-transitory recording medium may be in any form, and examples thereof include magnetic disks, optical disks, magneto-optical disks, and semiconductor memories.

The recording medium thus configured is capable of achieving the same actions and effects as those of the ophthalmic observation apparatus 1 and the ophthalmic image processing apparatus 500 of the above embodiment examples. In addition, by combining any of the matters and items relating to the ophthalmic observation apparatus 1 or the ophthalmic image processing apparatus 500 of the above embodiment examples with the recording medium, the resulting recording medium is capable of achieving the actions and effects corresponding to the combined matters and/or items.

## Claims

1. An ophthalmic observation apparatus for observing a subject's eye, comprising:
a moving image generating unit (10) configured to photograph the subject's eye to generate a moving image, in the following referred to as first moving image;
an image processor (211, 503) configured to create a plurality of processed images by applying first image processing using a plurality of different values of a predetermined image parameter to a still image included in the first moving image;
a display controller (201, 502) configured to display the plurality of processed images on a first display device (3, 504); and
an instruction receiving unit configured to receive an instruction for selecting at least one processed image from among the plurality of processed images displayed on the first display device (3, 504), **characterized in that**
the image processor (211, 503) is configured to apply second image processing to a second moving image, the second image processing being image processing based on at least one value of the image parameter corresponding to the at least one processed image, and the second moving image being an image generated by the moving image generating unit after selection of the at least one processed image is performed using the instruction receiving unit, and
the display controller (201, 502) is configured to display the second moving image to which the second image processing has been applied on a second display device (3, 504);
the ophthalmic observation apparatus further comprises
a recording unit (212) configured to record the one value of the image parameter used in the second image processing; and
an attribute information acquiring unit (222) configured to acquire attribute information that indicates an attribute of medical practice for the subject's eye, wherein the recording unit is configured to record the one value of the image parameter in association with the attribute information acquired by the attribute information acquiring unit (222).

2. The ophthalmic observation apparatus of claim 1, wherein the image processor (211, 503) is configured to apply image processing to the second moving image as the second image processing if one processed image of the plurality of processed images is selected using the instruction receiving unit, the image processing being performed using one value of the image parameter corresponding to the one processed image.

3. The ophthalmic observation apparatus of claim 1, wherein the image processor (211, 503) is configured to apply image processing to the second moving image as the second image processing if two or more processed images of the plurality of processed images are selected using the instruction receiving unit, the image processing being performed using one value of the image parameter corresponding to one processed image of the two or more processed images.

4. The ophthalmic observation apparatus of claim 1, wherein if two or more processed images of the plurality of processed images are selected using the instruction receiving unit, the image processor (211, 503) is configured to determine one value based on two or more values of the image parameter respectively corresponding to the two or more processed images, and is configured to apply image processing using the one value to the second moving image as the second image processing.

5. The ophthalmic observation apparatus of any of claims 1 to 4, further comprising an identifier receiving unit configured to receive an identifier of a user, wherein the recording unit (212) is configured to record the one value of the image parameter in association with the identifier received by the identifier receiving unit.

6. The ophthalmic observation apparatus of any of claims 1 to 5, further comprising a selecting processor configured to select at least one value from among values of the image parameter recorded by the recording unit (212) in the past, wherein the image processor is configured to apply image processing based on the at least one value selected by the selecting processor to a third moving image generated by the moving image generating unit.

7. The ophthalmic observation apparatus of claim 6, wherein
the recording unit (212) is configured to record a photographing condition applied to generation of the second moving image, in association with the one value of the image parameter, and
the selecting processor further is configured to select a photographing condition that is associated with the at least one value selected by the selecting processor,
the ophthalmic observation apparatus further comprising a determining processor configured to determine a value of the image parameter based on the photographing condition and the at least one value both selected by the selecting processor, wherein
the image processor (211, 503) is configured to apply image processing using the value of the image parameter determined by the determining processor to the third moving image.

8. The ophthalmic observation apparatus of any of claims 1 to 7, wherein
the image processor (211, 503) is configured to apply the first image processing to a partial image that is a part of the still image included in the first moving image to create a plurality of processed partial images as the plurality of processed images, and
the display controller (201, 502) is configured to display a plurality of images respectively including the plurality of processed partial images on the first display device (3, 504).

9. The ophthalmic observation apparatus of claim 8, wherein the image processor (211, 503) includes a first partial image identifying processor configured to identify the partial image by applying segmentation for identifying an image of a predetermined site of the subject's eye to the still image included in the first moving image.

10. The ophthalmic observation apparatus of claim 9, wherein
the first partial image identifying processor is configured to apply the segmentation to the second moving image to sequentially identify a plurality of partial images of a plurality of still images included in the second moving image, and
the image processor (211, 503) is configured to apply the second image processing sequentially to the plurality of partial images identified from the plurality of still images included in the second moving image.

11. The ophthalmic observation apparatus of claim 8, wherein the display controller (201, 502) is configured to display the first moving image or a still image included in the first moving image on the first display device or the second display device (3, 504),
the ophthalmic observation apparatus further comprising a graphical user interface for designating a partial region in the first moving image displayed or a partial region in the still image included in the first moving image, wherein
the image processor (211, 503) is configured to determine the partial image based on the partial region designated using the graphical user interface.

12. The ophthalmic observation apparatus of claim 11, wherein
the image processor (211, 503) includes a second partial image identifying processor configured to sequentially identify a plurality of partial images, each corresponding to the partial region, of a plurality of still images included in the second moving image, and
the image processor is configured to apply the second image processing sequentially to the plurality of partial images identified from the plurality of still images included in the second moving image.

13. The ophthalmic observation apparatus of any of claims 1 to 12, wherein the display controller (201, 502) is configured to align and display two or more processed images of the plurality of processed images or thumbnails of the two or more processed images on the first display device (3, 504).

14. The ophthalmic observation apparatus of any of claims 1 to 12, wherein the display controller (201, 502) is configured to display two or more processed images of the plurality of processed images or thumbnails of the two or more processed images one-by-one on the first display device (3, 504).

15. The ophthalmic observation apparatus of any of claims 1 to 14, further comprising a monitoring processor configured to monitor a movement of the subject's eye, wherein the display controller (201, 502) is configured to change a display state of the plurality of processed images based on output from the monitoring processor.

16. The ophthalmic observation apparatus of any of claims 1 to 15, further comprising an abnormality detecting processor configured to detect an abnormality of the subject's eye, wherein the display controller (201, 502) is configured to change a display state of the plurality of processed images based on output from the abnormality detecting processor.

17. The ophthalmic observation apparatus of any of claims 1 to 16, wherein the image parameter includes one or more of a color tone parameter, a brightness parameter, a contrast parameter, a gain parameter, a gamma parameter, a color temperature parameter, a white balance parameter, an RGB balance parameter, a gray balance parameter, an edge enhancement parameter, a shadow enhancement parameter, a sharpening parameter, and a high dynamic range parameter.

18. A computer-implemented method of processing an ophthalmic image, the method when executed on a computer comprising
receiving a first moving image of a subject's eye;
creating a plurality of processed images by applying first image processing using a plurality of different values of a predetermined image parameter to a still image included in the first moving image;
displaying the plurality of processed images;
receiving an instruction for selecting at least one processed image from among the plurality of processed images displayed;
receiving a second moving image of the subject's eye after selection of the at least one processed image is performed based on the instruction;
applying second image processing based on at least one value of the image parameter corresponding to the at least one processed image to the second moving image; and
displaying the second moving image to which the second image processing has been applied;
recording the one value of the image parameter used in the second image processing; and
acquiring attribute information that indicates an attribute of medical practice for the subject's eye, wherein the one value of the image parameter is recorded in association with the attribute information.

19. A computer-readable non-transitory recording medium storing a program causing a computer to execute the method of claim 18.

## Patentansprüche

1. Ophthalmische Beobachtungsvorrichtung zum Beobachten eines Auges einer Person, die Folgendes umfasst:
eine Bewegtbilderzeugungseinheit (10), die so konfiguriert ist, dass sie das Auge der Person fotografiert und so ein Bewegtbild erzeugt, das nachfolgend als erstes Bewegtbild bezeichnet wird,
einen Bildprozessor (211, 503), der so konfiguriert ist, dass er mehrere verarbeitete Bilder generiert, indem er eine erste Bildverarbeitung unter Verwendung mehrerer verschiedener Werte eines vorgegebenen Bildparameters auf ein Standbild aus dem ersten Bewegtbild anwendet,
eine Anzeigesteuerung (201, 502), die so konfiguriert ist, dass sie die mehreren verarbeiteten Bilder auf einer ersten Anzeigeeinrichtung (3, 504) anzeigt, und
eine Anweisungsempfangseinheit, die so konfiguriert ist, dass sie eine Anweisung zum Auswählen mindestens eines verarbeiteten Bildes unter den mehreren auf der ersten Anzeigeeinrichtung (3, 504) angezeigten verarbeiteten Bildern empfängt,
**dadurch gekennzeichnet, dass**
der Bildprozessor (211, 503) so konfiguriert ist, dass er auf ein zweites Bewegtbild eine zweite Bildverarbeitung anwendet, wobei es sich bei der zweiten Bildverarbeitung um eine Bildverarbeitung handelt, die darauf basiert, dass mindestens ein Wert für den Bildparameter dem mindestens einen verarbeiteten Bild entspricht, und es sich bei dem zweiten Bewegtbild um ein Bild handelt, das nach dem Auswählen des mindestens einen verarbeiteten Bildes mithilfe der Anweisungsempfangseinheit von der Bewegtbilderzeugungseinheit erzeugt wird, und
die Anzeigesteuerung (201, 502) so konfiguriert ist, dass sie das zweite Bewegtbild, auf das die zweite Bildverarbeitung angewendet wurde, auf einer zweiten Anzeigeeinrichtung (3, 504) anzeigt,
die ophthalmische Beobachtungsvorrichtung ferner Folgendes umfasst:
eine Aufzeichnungseinheit (212), die so konfiguriert ist, dass sie den einen bei der zweiten Bildverarbeitung verwendeten Wert für den Bildparameter aufzeichnet, und
eine Attributangabenerfassungseinheit (222), die so konfiguriert ist, dass sie Attributangaben erfasst, die ein Attribut ärztlicher Praxis für das Auge der Person angeben, wobei die Aufzeichnungseinheit so konfiguriert ist, dass sie den einen Wert für den Bildparameter in Verbindung mit den von der Attributangabenerfassungseinheit (222) erfassten Attributangaben aufzeichnet.

2. Ophthalmische Beobachtungsvorrichtung nach Anspruch 1, wobei der Bildprozessor (211, 503) so konfiguriert ist, dass er auf das zweite Bewegtbild eine Bildverarbeitung als zweite Bildverarbeitung anwendet, wenn mithilfe der Anweisungsempfangseinheit ein verarbeitetes Bild unter den mehreren verarbeiteten Bildern ausgewählt wird, wobei die Bildverarbeitung unter Verwendung eines Wertes für den Bildparameter durchgeführt wird, der dem einen verarbeiteten Bild entspricht.

3. Ophthalmische Beobachtungsvorrichtung nach Anspruch 1, wobei der Bildprozessor (211, 503) so konfiguriert ist, dass er auf das zweite Bewegtbild eine Bildverarbeitung als zweite Bildverarbeitung anwendet, wenn mithilfe der Anweisungsempfangseinheit zwei oder mehr verarbeitete Bilder unter den mehreren verarbeiteten Bildern ausgewählt werden, wobei die Bildverarbeitung unter Verwendung eines Wertes für den Bildparameter durchgeführt wird, der einem unter den zwei oder mehr verarbeiteten Bildern entspricht.

4. Ophthalmische Beobachtungsvorrichtung nach Anspruch 1, wobei der Bildprozessor (211, 503) so konfiguriert ist, dass er, wenn mithilfe der Anweisungsempfangseinheit zwei oder mehr verarbeitete Bilder unter den mehreren verarbeiteten Bildern ausgewählt werden, auf der Grundlage von zwei oder mehr Werten für den Bildparameter, die jeweils den zwei oder mehr verarbeiteten Bildern entsprechen, einen Wert bestimmt und eine Bildverarbeitung unter Verwendung des einen Werts als zweite Bildverarbeitung auf das zweite Bewegtbild anwendet.

5. Ophthalmische Beobachtungsvorrichtung nach einem der Ansprüche 1 bis 4, die ferner eine Kennungsempfangseinheit umfasst, die so konfiguriert ist, dass sie eine Kennung eines Benutzers empfängt, wobei die Aufzeichnungseinheit (212) so konfiguriert ist, dass sie den einen Wert für den Bildparameter in Verbindung mit der von der Kennungsempfangseinheit empfangenen Kennung aufzeichnet.

6. Ophthalmische Beobachtungsvorrichtung nach einem der Ansprüche 1 bis 5, die ferner einen Auswahlprozessor umfasst, der so konfiguriert ist, dass er unter von der Aufzeichnungseinheit (212) bereits aufgezeichneten Werten für den Bildparameter mindestens einen Wert auswählt, wobei der Bildprozessor so konfiguriert ist, dass er eine Bildverarbeitung auf der Grundlage des mindestens einen vom Auswahlprozessor ausgewählten Wertes auf ein von der Bewegtbilderzeugungseinheit erzeugtes drittes Bewegtbild anwendet.

7. Ophthalmische Beobachtungsvorrichtung nach Anspruch 6, wobei
die Aufzeichnungseinheit (212) so konfiguriert ist, dass sie eine Fotografierbedingung, die auf die Erzeugung des zweiten Bewegtbildes angewendet wird, in Verbindung mit dem einen Wert für den Bildparameter aufzeichnet, und
der Auswahlprozessor ferner so konfiguriert ist, dass er eine Fotografierbedingung auswählt, die mit dem mindestens einen vom Auswahlprozessor ausgewählten Wert verbunden ist,
die ophthalmische Beobachtungsvorrichtung ferner einen Bestimmungsprozessor umfasst, der so konfiguriert ist, dass er auf der Grundlage der Fotografierbedingung und des mindestens einen Wertes, die beide vom Auswahlprozessor ausgewählt wurden, einen Wert für den Bildparameter bestimmt, wobei
der Bildprozessor (211, 503) so konfiguriert ist, dass er eine Bildverarbeitung unter Verwendung des vom Bestimmungsprozessor bestimmten Wertes für den Bildparameter auf das dritte Bewegtbild anwendet.

8. Ophthalmische Beobachtungsvorrichtung nach einem der Ansprüche 1 bis 7, wobei
der Bildprozessor (211, 503) so konfiguriert ist, dass er die erste Bildverarbeitung auf ein Teilbild anwendet, das zu dem Standbild aus dem ersten Bewegtbild gehört, und so mehrere verarbeitete Teilbilder als die mehreren verarbeiteten Bilder generiert, und
die Anzeigesteuerung (201, 502) so konfiguriert ist, dass sie mehrere Bilder, zu denen jeweils die mehreren verarbeiteten Teilbilder gehören, auf der ersten Anzeigeeinrichtung (3, 504) anzeigt.

9. Ophthalmische Beobachtungsvorrichtung nach Anspruch 8, wobei der Bildprozessor (211, 503) einen ersten Teilbilderkennungsprozessor aufweist, der so konfiguriert ist, dass er durch Anwenden einer Segmentierung zum Erkennen eines Bildes von einer vorgegebenen Lokalisation im Auge der Person auf das Standbild aus dem ersten Bewegtbild das Teilbild erkennt.

10. Ophthalmische Beobachtungsvorrichtung nach Anspruch 9, wobei
der erste Teilbilderkennungsprozessor so konfiguriert ist, dass er die Segmentierung auf das zweite Bewegtbild anwendet und so nacheinander mehrere Teilbilder von mehreren Standbildern aus dem zweiten Bewegtbild erkennt, und
der Bildprozessor (211, 503) so konfiguriert ist, dass er die zweite Bildverarbeitung nacheinander auf die mehreren Teilbilder anwendet, die in den mehreren Standbildern aus dem zweiten Bewegtbild erkannt wurden.

11. Ophthalmische Beobachtungsvorrichtung nach Anspruch 8, wobei
die Anzeigesteuerung (201, 502) so konfiguriert ist, dass sie das erste Bewegtbild oder ein Standbild aus dem ersten Bewegtbild auf der ersten oder der zweiten Anzeigeeinrichtung (3, 504) anzeigt,
die ophthalmische Beobachtungsvorrichtung ferner eine grafische Benutzerschnittstelle zum Kennzeichnen einer Teilregion im ersten angezeigten Bewegtbild oder einer Teilregion im Standbild aus dem ersten Bewegtbild umfasst, wobei
der Bildprozessor (211, 503) so konfiguriert ist, dass er das Teilbild auf der Grundlage der mithilfe der grafischen Benutzerschnittstelle gekennzeichneten Teilregion bestimmt.

12. Ophthalmische Beobachtungsvorrichtung nach Anspruch 11, wobei
der Bildprozessor (211, 503) einen zweiten Teilbilderkennungsprozessor aufweist, der so konfiguriert ist, dass er nacheinander mehrere jeweils der Teilregion entsprechende Teilbilder von mehreren Standbildern aus dem zweiten Bewegtbild erkennt, und
der Bildprozessor so konfiguriert ist, dass er die zweite Bildverarbeitung nacheinander auf die mehreren Teilbilder anwendet, die in den mehreren Standbildern aus dem zweiten Bewegtbild erkannt wurden.

13. Ophthalmische Beobachtungsvorrichtung nach einem der Ansprüche 1 bis 12, wobei die Anzeigesteuerung (201, 502) so konfiguriert ist, dass sie zwei oder mehr verarbeitete Bilder unter den verarbeiteten Bildern oder Miniaturbilder von den zwei oder mehr verarbeiteten Bildern auf der ersten Anzeigeeinrichtung (3, 504) ausrichtet und anzeigt.

14. Ophthalmische Beobachtungsvorrichtung nach einem der Ansprüche 1 bis 12, wobei die Anzeigesteuerung (201, 502) so konfiguriert ist, dass sie zwei oder mehr verarbeitete Bilder unter den verarbeiteten Bildern oder Miniaturbilder von den zwei oder mehr verarbeiteten Bildern eins nach dem anderen auf der ersten Anzeigeeinrichtung (3, 504) anzeigt.

15. Ophthalmische Beobachtungsvorrichtung nach einem der Ansprüche 1 bis 14, die ferner einen Überwachungsprozessor umfasst, der so konfiguriert ist, dass er eine Bewegung des Auges der Person überwacht, wobei die Anzeigesteuerung (201, 502) so konfiguriert ist, dass sie einen Anzeigezustand der mehreren verarbeiteten Bilder auf der Grundlage einer Ausgabe des Überwachungsprozessors ändert.

16. Ophthalmische Beobachtungsvorrichtung nach einem der Ansprüche 1 bis 15, die ferner einen Anomaliedetektionsprozessor umfasst, der so konfiguriert ist, dass er eine Anomalie im Auge der Person detektiert, wobei die Anzeigesteuerung (201, 502) so konfiguriert ist, dass sie einen Anzeigezustand der mehreren verarbeiteten Bilder auf der Grundlage einer Ausgabe des Anomaliedetektionsprozessors ändert.

17. Ophthalmische Beobachtungsvorrichtung nach einem der Ansprüche 1 bis 16, wobei der Bildparameter einen oder mehrere der folgenden Parameter umfasst: einen Farbtonparameter, einen Helligkeitsparameter, einen Kontrastparameter, einen Verstärkungsparameter, einen Gamma-Parameter, einen Farbtemperaturparameter, einen Weißabgleichparameter, einen RGB-Abgleichparameter, einen Grauabgleichparameter, einen Kantenschärfungsparameter, einen Schattenoptimierungsparameter, einen Scharfzeichnungsparameter und einen Parameter für einen hohen Dynamikumfang.

18. Computergestütztes Verfahren zum Verarbeiten eines ophthalmischen Bildes, wobei das Verfahren, wenn es auf einem Computer ausgeführt wird, Folgendes umfasst:
Empfangen eines ersten Bewegtbildes vom Auge einer Person,
Generieren mehrerer verarbeiteter Bilder durch Anwenden einer ersten Bildverarbeitung auf ein Standbild aus dem ersten Bewegtbild unter Verwendung mehrerer verschiedener Werte für einen vorgegebenen Bildparameter,
Anzeigen der mehreren verarbeiteten Bilder,
Empfangen einer Anweisung zum Auswählen mindestens eines verarbeiteten Bildes unter den mehreren angezeigten verarbeiteten Bildern,
Empfangen eines zweiten Bewegtbildes vom Auge der Person nach dem Auswählen des mindestens einen verarbeiteten Bildes auf der Grundlage der Anweisung,
Anwenden einer zweiten Bildverarbeitung, die darauf basiert, dass mindestens ein Wert für den Bildparameter dem mindestens einen verarbeiteten Bild entspricht, auf das zweite Bewegtbild und
Anzeigen des zweiten Bewegtbildes, auf das die zweite Bildverarbeitung angewendet wurde,
Aufzeichnen des einen Wertes für den Bildparameter, der bei der zweiten Bildverarbeitung verwendet wurde, und
Erfassen von Attributangaben, die ein Attribut ärztlicher Praxis für das Auge der Person angeben, wobei der eine Wert für den Bildparameter in Verbindung mit den Attributangaben aufgezeichnet wird.

19. Computerlesbares, nichtflüchtiges Aufzeichnungsmedium, auf dem ein Programm gespeichert ist, das einen Computer dazu veranlasst, das Verfahren nach Anspruch 18 auszuführen.

## Revendications

1. Appareil d'observation ophtalmique pour observer l'œil d'un sujet, comprenant :
une unité de génération d'image animée (10) configurée pour photographier l'œil du sujet afin de générer une image animée, appelée ci-après première image animée ;
un processeur d'image (211, 503) configuré pour créer une pluralité d'images traitées par application d'un premier traitement d'image utilisant une pluralité de valeurs différentes d'un paramètre d'image prédéterminé à une image fixe incluse dans la première image animée ;
un contrôleur d'affichage (201, 502) configuré pour afficher la pluralité d'images traitées sur un premier dispositif d'affichage (3, 504) ; et
une unité de réception d'instruction configurée pour recevoir une instruction pour sélectionner au moins une image traitée parmi la pluralité d'images traitées affichées sur le premier dispositif d'affichage (3, 504),
**caractérisé en ce que**
le processeur d'image (211, 503) étant configuré pour appliquer un second traitement d'image à une deuxième image animée, le second traitement d'image étant un traitement d'image basé sur au moins une valeur du paramètre d'image correspondant à l'au moins une image traitée, et la deuxième image animée étant une image générée par l'unité de génération d'image animée après que la sélection de l'image traitée a été effectuée à l'aide de l'unité de réception d'instruction, et
le contrôleur d'affichage (201, 502) étant configuré pour afficher la deuxième image animée à laquelle le second traitement d'image a été appliqué sur un second dispositif d'affichage (3, 504) ;
l'appareil d'observation ophtalmique comprenant en outre :
une unité d'enregistrement (212) configurée pour enregistrer la valeur du paramètre d'image utilisé dans le second traitement d'image ; et
une unité d'acquisition d'informations d'attribut (222) configurée pour acquérir des informations d'attribut qui indiquent un attribut de pratique médicale pour l'œil du sujet, l'unité d'enregistrement étant configurée pour enregistrer la valeur du paramètre d'image en association avec les informations d'attribut acquises par l'unité d'acquisition d'informations d'attribut (222).

2. Appareil d'observation ophtalmique selon la revendication 1, dans lequel le processeur d'image (211, 503) est configuré pour appliquer un traitement d'image à la deuxième image animée en tant que second traitement d'image si une image traitée de la pluralité d'images traitées est sélectionnée en utilisant l'unité de réception d'instructions, le traitement d'image étant effectué en utilisant une valeur du paramètre d'image correspondant à l'image traitée.

3. Appareil d'observation ophtalmique selon la revendication 1, dans lequel le processeur d'image (211, 503) est configuré pour appliquer un traitement d'image à la deuxième image animée en tant que second traitement d'image si deux images traitées ou plus de la pluralité d'images sélectionnées en utilisant l'unité de réception d'instruction, le traitement d'image étant effectué en utilisant une valeur du paramètre d'image correspondant à une image traitée des deux images traitées ou plus.

4. Appareil d'observation ophtalmique selon la revendication 1, dans lequel, si deux ou plusieurs images traitées de la pluralité d'images traitées sont sélectionnées en utilisant l'unité de réception d'instruction, le processeur d'image (211, 503) est configuré pour déterminer une valeur basée sur deux ou plusieurs valeurs du paramètre d'image correspondant respectivement aux deux ou plusieurs images traitées, et est configuré pour appliquer un traitement d'image en utilisant la valeur de la deuxième image animée en tant que second traitement d'image.

5. Appareil d'observation ophtalmique selon l'une quelconque des revendications 1 à 4, comprenant en outre une unité de réception d'identifiant configurée pour recevoir un identifiant d'un utilisateur, l'unité d'enregistrement (212) étant configurée pour enregistrer la valeur du paramètre d'image en association avec l'identifiant reçu par l'unité de réception d'identifiant.

6. Appareil d'observation ophtalmique selon l'une quelconque des revendications 1 à 5, comprenant en outre un processeur de sélection configuré pour sélectionner au moins une valeur parmi les valeurs du paramètre d'image enregistrées par l'unité d'enregistrement (212) dans le passé, le processeur d'image étant configuré pour appliquer un traitement d'image basé sur la au moins une valeur sélectionnée par le processeur de sélection à une troisième image animée générée par l'unité de génération d'image animée.

7. Appareil d'observation ophtalmique selon la revendication 6, dans lequel
l'unité d'enregistrement (212) est configurée pour enregistrer une condition de photographie appliquée à la génération de la deuxième image animée, en association avec la première valeur du paramètre d'image, et
le processeur de sélection est en outre configuré pour sélectionner une condition de photographie associée à au moins une valeur sélectionnée par le processeur de sélection,
l'appareil d'observation ophtalmique comprend en outre un processeur de détermination configuré pour déterminer une valeur du paramètre d'image sur la base de la condition de photographie et de l'au moins une valeur sélectionnée par le processeur de sélection, le processeur d'image (211, 503) étant configuré pour appliquer un traitement d'image à la troisième image animée en utilisant la valeur du paramètre d'image déterminée par le processeur de détermination.

8. Appareil d'observation ophtalmique selon l'une quelconque des revendications 1 à 7, dans lequel
le processeur d'image (211, 503) est configuré pour appliquer le premier traitement d'image à une image partielle qui est une partie de l'image fixe incluse dans la première image animée pour créer une pluralité d'images partielles traitées en tant que pluralité d'images traitées, et
le contrôleur d'affichage (201, 502) est configuré pour afficher une pluralité d'images comprenant respectivement la pluralité d'images partielles traitées sur le premier dispositif d'affichage (3, 504).

9. Appareil d'observation ophtalmique selon la revendication 8, dans lequel le processeur d'image (211, 503) comprend un premier processeur d'identification d'image partielle configuré pour identifier l'image partielle en appliquant une segmentation pour identifier une image d'un site prédéterminé de l'œil du sujet à l'image fixe incluse dans la première image animée.

10. Appareil d'observation ophtalmique selon la revendication 9, dans lequel
le premier processeur d'identification d'image partielle est configuré pour appliquer la segmentation à la deuxième image animée afin d'identifier séquentiellement une pluralité d'images partielles d'une pluralité d'images fixes comprises dans la deuxième image animée, et
le processeur d'image (211, 503) est configuré pour appliquer le second traitement d'image séquentiellement à la pluralité d'images partielles identifiées à partir de la pluralité d'images fixes incluses dans la deuxième image animée.

11. Appareil d'observation ophtalmique selon la revendication 8, dans lequel
le contrôleur d'affichage (201, 502) est configuré pour afficher la première image animée ou une image fixe incluse dans la première image animée sur le premier dispositif d'affichage ou le second dispositif d'affichage (3, 504),
l'appareil d'observation ophtalmique comprend en outre une interface utilisateur graphique pour désigner une zone partielle dans la première image animée affichée ou une zone partielle dans l'image fixe incluse dans la première image animée,
le processeur d'image (211, 503) étant configuré pour déterminer l'image partielle sur la base de la zone partielle désignée au moyen de l'interface utilisateur graphique.

12. Appareil d'observation ophtalmique selon la revendication 11, dans lequel
le processeur d'image (211, 503) comprend un second processeur d'identification d'image partielle configuré pour identifier séquentiellement une pluralité d'images partielles, chacune correspondant à la zone partielle, d'une pluralité d'images fixes comprises dans la deuxième image animée, et
le processeur d'image est configuré pour appliquer le second traitement d'image séquentiellement à la pluralité d'images partielles identifiées à partir de la pluralité d'images fixes incluses dans la deuxième image animée.

13. Appareil d'observation ophtalmique selon l'une quelconque des revendications 1 à 12, dans lequel le contrôleur d'affichage (201, 502) est configuré pour aligner et afficher deux ou plusieurs images traitées de la pluralité d'images traitées ou des vignettes des deux ou plusieurs images traitées sur le premier dispositif d'affichage (3, 504).

14. Appareil d'observation ophtalmique selon l'une quelconque des revendications 1 à 12, dans lequel le contrôleur d'affichage (201, 502) est configuré pour afficher deux ou plusieurs images traitées de la pluralité d'images traitées ou des vignettes des deux ou plusieurs images traitées une par une sur le premier dispositif d'affichage (3, 504).

15. Appareil d'observation ophtalmique selon l'une quelconque des revendications 1 à 14, comprenant en outre un processeur de surveillance configuré pour surveiller un mouvement de l'œil du sujet, le contrôleur d'affichage (201, 502) étant configuré pour changer un état d'affichage de la pluralité d'images traitées sur la base d'une sortie du processeur de surveillance.

16. Appareil d'observation ophtalmique selon l'une quelconque des revendications 1 à 15, comprenant en outre un processeur de détection d'anomalie configuré pour détecter une anomalie de l'œil du sujet, le contrôleur d'affichage (201, 502) étant configuré pour changer un état d'affichage de la pluralité d'images traitées sur la base d'une sortie du processeur de détection d'anomalie.

17. Appareil d'observation ophtalmique selon l'une quelconque des revendications 1 à 16, dans lequel le paramètre d'image comprend un ou plusieurs parmi un paramètre de tonalité de couleur, un paramètre de luminosité, un paramètre de contraste, un paramètre de gain, un paramètre gamma, un paramètre de température de couleur, un paramètre d'équilibre des blancs, un paramètre d'équilibre RVB, un paramètre d'équilibre des gris, un paramètre d'amélioration des contours, un paramètre d'amélioration des ombres, un paramètre d'accentuation de la netteté et un paramètre de plage dynamique élevée.

18. Procédé informatisé de traitement d'une image ophtalmique, le procédé, lorsqu'il est exécuté sur un ordinateur, comprenant
la réception d'une première image animée de l'œil d'un sujet ;
la création d'une pluralité d'images traitées par application d'un premier traitement d'image en utilisant une pluralité de valeurs différentes d'un paramètre d'image prédéterminé d'une image fixe incluse dans la première image animée ;
l'affichage de la pluralité d'images traitées ;
la réception d'une instruction de sélection d'au moins une image traitée parmi la pluralité d'images traitées affichées ;
la réception d'une seconde image animée de l'œil du sujet après que la sélection de l'au moins une image traitée a été effectuée sur la base de l'instruction ;
l'application d'un second traitement d'image sur la base d'au moins une valeur du paramètre d'image correspondant à l'au moins une image traitée à la deuxième image animée ; et
l'affichage de la deuxième image animée à laquelle le second traitement d'image a été appliqué ;
l'enregistrement de la valeur du paramètre d'image utilisé dans le second traitement d'image ; et
l'acquisition d'informations d'attribut indiquant un attribut de pratique médicale pour l'œil du sujet, la valeur du paramètre d'image étant enregistrée en association avec les informations d'attribut.

19. Support d'enregistrement non transitoire lisible par ordinateur stockant un programme amenant un ordinateur à exécuter le procédé selon la revendication 18.
